# EUROPEAN PATENT SPECIFICATION

(11) **EP 3 717 978 B1**
(45) Date of publication and mention of the grant of the patent: **14.01.2026**
(21) Application number: 18882550.9
(22) Date of filing: 30.11.2018
(51) Int. Cl.: G05B 19/418, G16C 10/00, G06Q 10/10, G16H 20/10, G16H 40/20

(54) **COMPOUNDING DEVICE SYSTEM**
COMPOUNDIERVORRICHTUNGSSYSTEM
SYSTÈME DE DISPOSITIF DE MÉLANGE

(30) Priority: 30.11.2017 US 201762592609 P
(43) Date of publication of application: 07.10.2020
(73) Proprietor: Omnicell, Inc., Mountain View, CA 94043 (US)
(72) Inventor: MARSH, Charles, Cranberry Township Pennsylvania 16066 (US); MCCUTCHAN, Larry, Cranberry Township Pennsylvania 16066 (US)
(74) Representative: Bird & Bird LLP
(86) International application number: PCT/US2018/063493
(87) International publication number: WO 2019/109048

(56) References cited:
- WO-A1-03/058507
- WO-A1-2014/123147
- US-A1- 2007 088 590
- US-A1- 2008 195 246
- US-A1- 2016 232 325
- US-B1- 6 711 460

## Description

### CROSS REFERENCE TO RELATED APPLICATIONS

This application claims the benefit of Provisional U.S. Patent Application No. 62/592,609 filed November 30, 2017 and titled "Compounding Device System,

### BACKGROUND OF THE INVENTION

Pharmaceutical compounding is the preparation of patient-specific medications by the processing or combination of ingredients. Many medications, especially medications administered orally in pill form, are now manufactured in a variety of forms and dosages so that little preparation is needed at a pharmacy, other than placing the proper number of pills in a bottle to fill a doctor's prescription for a particular patient. However, medications for intravenous delivery are routinely compounded, for example in hospital pharmacies.

Typically, a physician will prescribe a particular medication or a combination of medications for a specific patient, for intravenous (IV) delivery. The pharmacy receives the prescription and prepares the IV solution with the proper amount of each prescribed medication. The compounded medication is then sent to the hospital floor for administration to the patient.

It is of utmost importance that the correct medications be prepared in the correct proportions, without the introduction of contaminants. Detailed protocols may be developed for the compounder to follow. The number of different protocols may be very large, because there may be a large number of different medications to choose from, in a variety of packages, to be prepared in a number of dosages, and to be provided in a number of different delivery vehicles.

Much of the work of compounding may be delegated to workers who are not registered pharmacists, or to robotic machines. Accordingly meticulous records may be kept of the preparation of each medication, so that the pharmacist can review how each medication was made before it leaves the pharmacy. The records also enable review of the preparation of any particular medication at a later time, should there be any question of its correctness.

In addition, it is desirable that pharmacy resources be used efficiently, and that excessive waste of medications and supplies be avoided.

US 6 711 460 B1 discloses a pharmaceutical system and method of operation in which a single remote professional provides pharmaceutical care and oversight of multiple local pharmacies. A control location is connected through an electronic network to one or more individual pharmacies, each of which may be located at a different physical site. Each individual pharmacy includes one or more drug preparation areas, and one or more self-service or staffed customer terminals. A drug preparation area includes a robot, which is adapted to prepare prescriptions or other items, and which is connected by a pneumatic delivery system to one or more customer terminals within the pharmacy.

### BRIEF SUMMARY OF THE INVENTION

The invention is set out in the appended set of claims.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1 illustrates a compounding pharmacy in accordance with embodiments of the invention.
FIG. 2 illustrates a manual compounding station in accordance with embodiments of the invention.
FIG. 3 shows a compounding assistance device, in accordance with embodiments of the invention.
FIG. 4 shows a lower oblique view of the compounding assistance device of FIG. 3, in accordance with embodiments of the invention.
FIG. 5 illustrates bar code scanning by the compounding assistance device of FIG. 3, in accordance with embodiments of the invention.
FIG. 6 illustrates a step in a compounding process, in accordance with embodiments of the invention.
FIG. 7 illustrates another step in the compounding process, in accordance with embodiments of the invention.
FIG. 8 illustrates another step in the compounding process, in accordance with embodiments of the invention.
FIG. 9 illustrates another step in the compounding process, in accordance with embodiments of the invention.
FIG. 10 shows a photograph as may be taken using a visible light camera, in accordance with embodiments of the invention.
FIG. 11 shows a photograph of a syringe as may be taken using an infrared camera, in accordance with embodiments of the invention.
FIG. 12 illustrates another step in the compounding process, in accordance with embodiments of the invention.
FIG. 13 illustrates another step in the compounding process, in accordance with embodiments of the invention.
FIG. 14 illustrates the arrangement of an area light source and an infrared camera of the compounding assistance device of FIG. 3, in an embodiment of the invention.
FIG. 15 shows a photograph similar to the photograph of FIG. 11, taken using an infrared camera, and illustrating image analysis in accordance with embodiments of the invention.
FIG. 16 illustrates an example of a trace of the brightness of pixels along a column line in the image of FIG. 15, in accordance with embodiments of the invention.
FIG. 17 illustrates a way of annotating an infrared image, in accordance with embodiments of the invention.
FIG. 18 illustrates the arrangement of a visible light camera in the compounding assistance device of FIG. 3, in an embodiment of the invention.
FIG. 19 shows an example photograph of a number of syringes, as may be taken by a visible light camera in accordance with embodiments of the invention.
FIG. 20 illustrates a simplified block diagram of the compounding assistance device of FIG. 3, in accordance with embodiments of the invention.
FIG. 21 schematically shows one example arrangement of multiple pharmacies in multiple facilities, all in communication with a pharmacy server, in accordance with embodiments of the invention.
FIG. 22 illustrates a high-level block diagram of a resource control system, in accordance with disclosed embodiments.
FIG. 23 shows a diagram of a portion of an architecture stack of the system if FIG. 22, in accordance with certain embodiments.

### DETAILED DESCRIPTION OF THE INVENTION

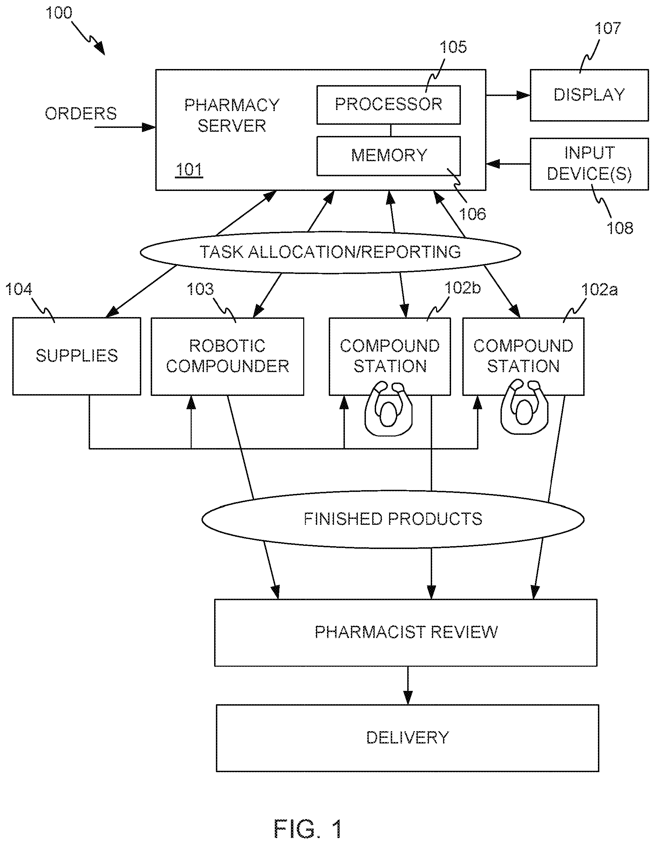

**FIG. 1** illustrates a compounding pharmacy **100** in accordance with embodiments of the invention. The operation of pharmacy **100** is coordinated by a pharmacy server **101,** described in more detail below. Pharmacy server **101** receives orders for compounded medications, for example prescriptions from physicians. Pharmacy server **101** maintains extensive records of orders received, detailed protocols for the compounding of medications, records of the preparation of medications in response to orders, and other items. Pharmacy server **101** also allocates tasks to one or more compounding stations, which may include manual compounding stations such as stations **102a** and **102b,** and one or more robotic compounders **103.** The compounding stations may also report information to pharmacy server **101**, for example records of the compounding of each ordered medication.

Pharmacy server **101** includes a processor **105** and memory **106.** Memory **106** holds instructions that, when executed by processor **105,** cause pharmacy server to perform its functions in accordance with embodiments of the invention. Memory **106** may also hold the records, protocols, and other information collected and generated in the operation of pharmacy **100.** For the purposes of this disclosure, the term "memory" encompasses many different kinds of data storage devices and combinations of such devices, for example dynamic memory, static memory, volatile memory, nonvolatile memory, and mass storage such as magnetic or optical disk storage or tape storage.

While pharmacy server **101** is shown as a single block in FIG. 1 and could be a single, stand-alone computer system having memory **106** and one or more processors **105,** other implementations are possible. For example, pharmacy server may be implemented using a number of interconnected computers, either co-located or in multiple locations. In particular, pharmacy server **101** may be implemented as a "cloud" service, in which the functions of pharmacy server **101** may be performed by different processors at different times, and memory **106** may be distributed as well. Pharmacy server **101** presents information to a user via a user interface shown on an electronic display **107,** and may receive inputs from the user via any input device or devices **108,** for example a keyboard, mouse, other pointing device, or other input devices or combinations of input devices.

Working materials are supplied to the compounding stations from a supply store **104.** Pharmacy server **101** may maintain an inventory of the materials in supply store **104,** and may track the movements of medications and supplies within pharmacy **100.**

Finished products are reviewed by the pharmacist and delivered from pharmacy **100** to their points of use, for example patient rooms for administration by a nurse to a patient. It will be understood that the above description is highly generalized, and that a working compounding pharmacy may have many other systems and facilities.

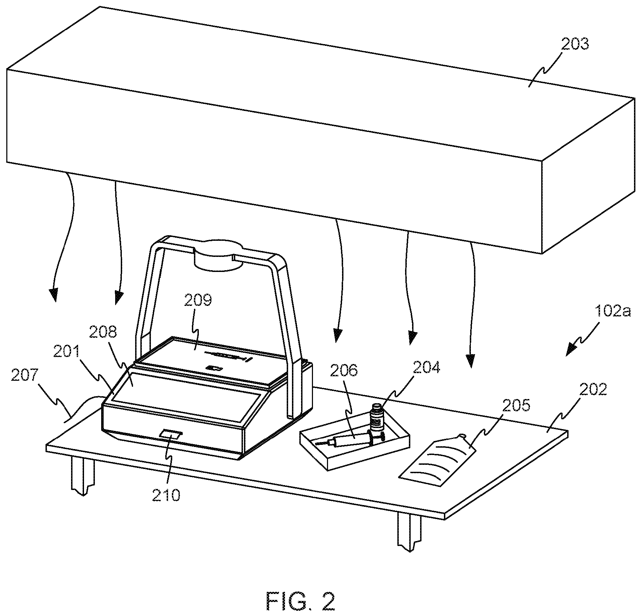

**FIG. 2** illustrates a manual compounding station **102a** in accordance with embodiments of the invention. Compounding station **102a** includes a compounding assistance device **201** on a surface **202.** For the purposes of this disclosure, a compounding assistance device is an electromechanical device having features and capabilities for facilitating the performance of a compounding task by a human operator. Compounding assistance device **201** may be placed under a laminar flow hood **203,** which flows filtered air over compounding assistance device **201** and surface **202,** to help avoid contamination of the materials being worked on, and for protection of the user of compounding station **102a.**

In the example shown, compounding station **102a** has received supplies for a simple compounding task. A medication supplied in a vial **204** is to be added to an IV drip bag **205.** A syringe **206** may be used to accomplish the transfer.

Compounding assistance device **201** has several features and capabilities that will assist the compounder in properly preparing the formulation in IV drip bag **205,** and in thoroughly documenting the process. Compounding assistance device **201** has a network connection **207** to pharmacy server **101,** though which compounding assistance device **201** may receive a protocol from pharmacy server **101** describing the steps required to perform the compounding task.

For the purposes of this disclosure, a protocol is a sequence of steps for a specific compounding task involving a specific medication. A workflow is a generic set of steps, specified independent of the particular medication and dosage of the specific compounding task. One workflow can describe the generic steps required for a kind of compounding task. Many different protocols may implement the workflow, for specific medications and amounts. For example, a particular workflow may describe the high level steps needed to draw medication from a vial and add it to an IV drip bag. Separate protocols can then describe the steps for placing a specific dosage of a specific medication in the drip bag.

Compounding assistance device **201** includes a display screen **208** on which instructions to the user may be presented or through which the user may input information. For example, display screen **208** may be a touchscreen display, sensitive to touch and able to distinguish the location of a touch. Compounding assistance device **201** also includes a tray **209** which provides a carrier for holding items while they are weighed or photographed, as is described in more detail below.

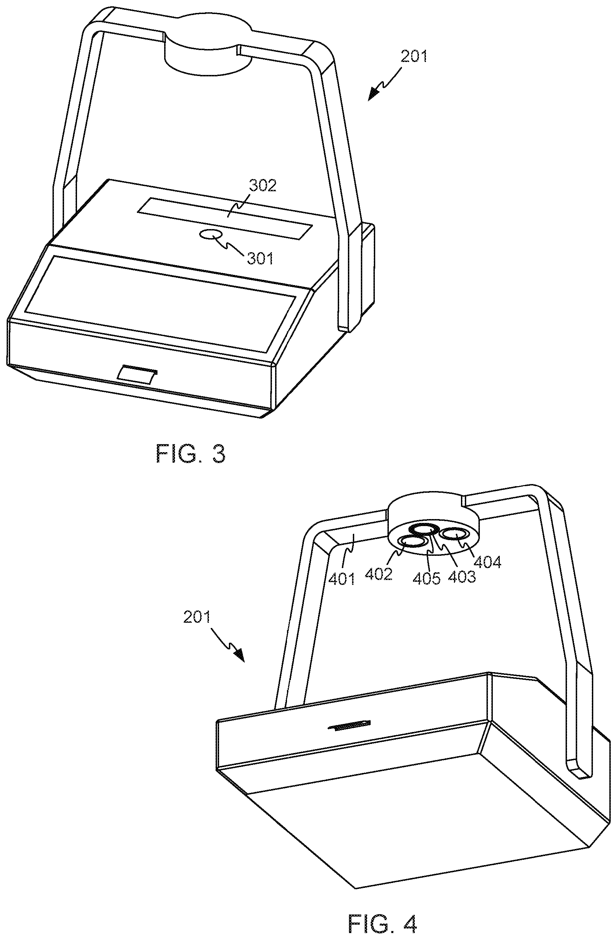

**FIG. 3** shows compounding assistance device **201,** with tray **209** removed, in accordance with embodiments of the invention. Visible in FIG. 3 is a weight sensor **301,** for example a load cell, for weighing tray **209** and its contents. Also visible is an area light source **302.** Area light source **302** is a two-dimensional extended or area light source, and emits light from many points or continuously across its face. Area light source **302** may be, for example, an infrared light panel, illuminating a portion of tray **209** from below with infrared light.

**FIG. 4** shows a lower oblique view of compounding assistance device **201,** in accordance with embodiments of the invention. A gantry **401** spans tray **209.** Positioned on gantry **401** are a bar code scanner **402,** a visible light camera **403,** and an infrared camera **404**. Visible light camera **403** may further include one or more light sources **405** for illuminating at least a portion of tray **209** from above. Light sources **405** may be, for example, one or more white-light light emitting diodes (LEDs) surrounding visible light camera **403,** or another kind of light source. For the purposes of this disclosure, light is "visible" if it includes light wavelengths between about 400 and 700 nanometers. Light is "white" if it includes enough wavelengths in the visible range to enable reasonably complete color recognition.

The area above tray **209** may be called a viewing area for items to be photographed by infrared camera **404** or visible light camera **403,** or scanned by bar code scanner **402.** In other embodiments, an item may not necessarily be lit from below and photographed from above. For example, in a compounding robot, a robotic mechanism may hold an item to be photographed in the field of view of a camera in any orientation. For example, an item may be photographed from below, or horizontally.

Bar code scanner **402** is positioned to read bar codes on items held in the viewing area between tray **209** and bar code scanner 402. Visible light camera **403** and infrared camera **404** are position to take photographs of items on tray **209.**

During compounding of a medication one or more of weight sensor **301,** bar code scanner **402,** visible light camera **403,** and infrared camera **404** can be used to provide documentation of how the medication was compounded, and to avoid errors.

For example, to perform the compounding task illustrated in FIG. 2, pharmacy server **101** sends detailed sequential instructions to compounding assistance device **201,** which then leads the user through the steps required to formulate the specific medication in the specific dose required, for delivery in the specific delivery vehicle. In this example, the task may involve transferring 30000 units of Heparin (a common anticoagulant) from a vial containing 5000 units/ml of Heparin in solution, to an IV drip bag. The volume of solution required for transfer is therefore 6 ml. Vial **204** and IV drip bag **205** have been supplied to compounding station **102a,** along with syringe 206, which will be needed to make the transfer.

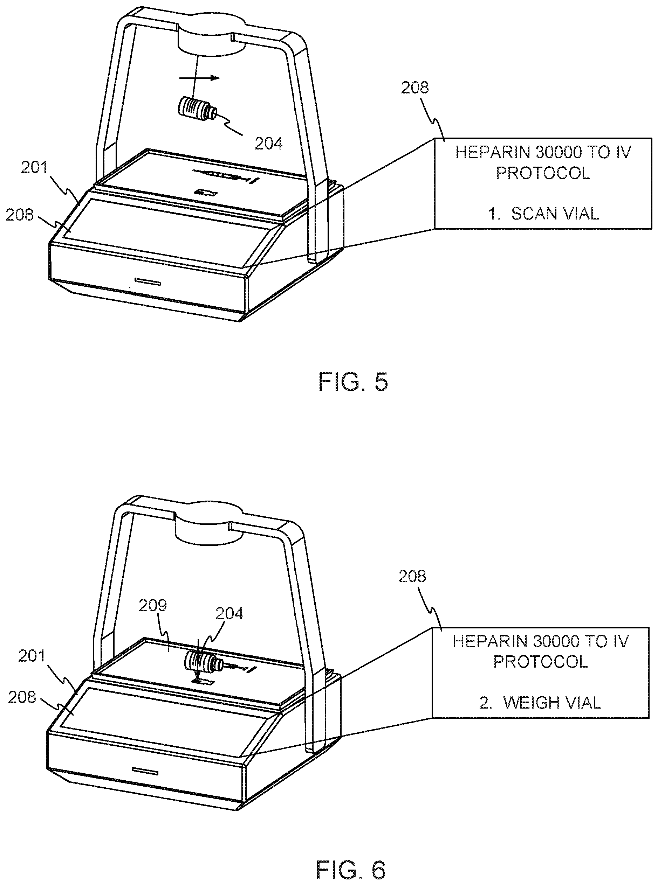

First, compounding assistance device **201** requires that the user present vial 204 to bar code scanner **402,** so that the identifying bar code on vial **204** can be read, and the system can verify that the correct vial with the correct concentration has been provided. If not, then an error message is generated and the compounding task is stopped. The scanning process is illustrated in **FIG. 5****,** along with an example prompt shown on screen **208.** Compounding assistance device **201** may automatically recognize that the barcode has been detected, and may move to the next step. Alternatively, an acknowledgment from the user may be required, in this and other steps.

**FIG.** 6 illustrates a second step in the compounding process, in which an initial weight of vial **204** is collected. For this purpose, vial **204** is placed on tray **209.** Tray **209** may include an icon **601** indicating where vial **204** should be placed, and may also include mechanical features for aiding in proper placement of vial **204.** For example, a gently V-shaped trough may be formed into tray **209.** Compounding assistance device **201** may automatically recognize the weight of vial **204** on tray **209,** record the weight, and move to then next step of the compounding process.

In some embodiments, vial **204** may also be photographed while on tray **209** using visible light camera **403,** using ambient light, light from light sources **405,** or a combination thereof.

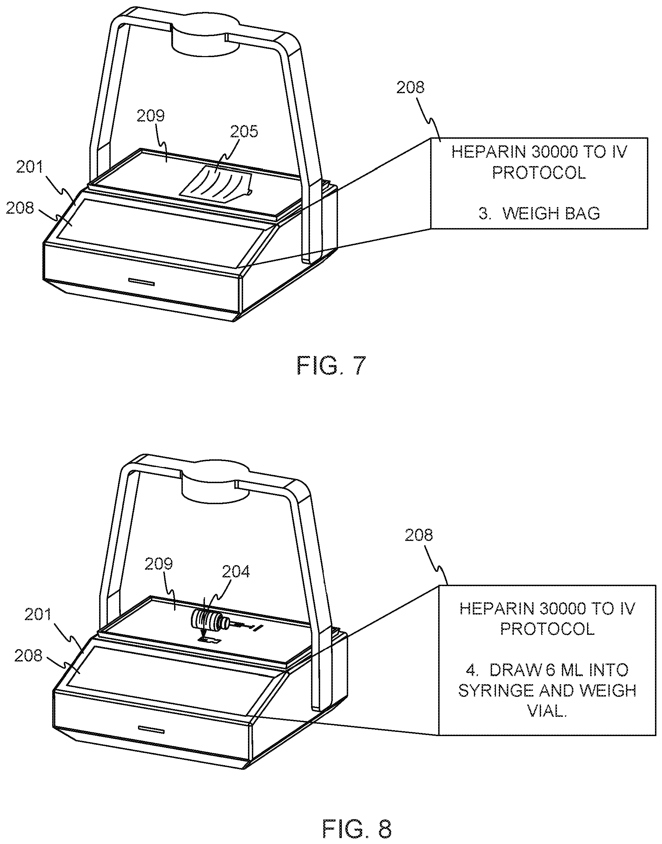

**FIG. 7** illustrates a third step, in which an initial weight of IV bag **205** is collected. Compounding assistance device **201** may then prompt the user to draw the correct amount (6 ml) of solution from vial **204** into syringe **206**.

**FIG. 8** illustrates a fourth step, in which an after-drawn weight of vial **204** is taken, in a manner similar to the taking of the initial vial weight shown in FIG. 6. The system can compare the two weights of vial **204** to calculate the amount of solution drawn from vial **204**, for recordkeeping and for verification that the proper amount of solution was drawn.

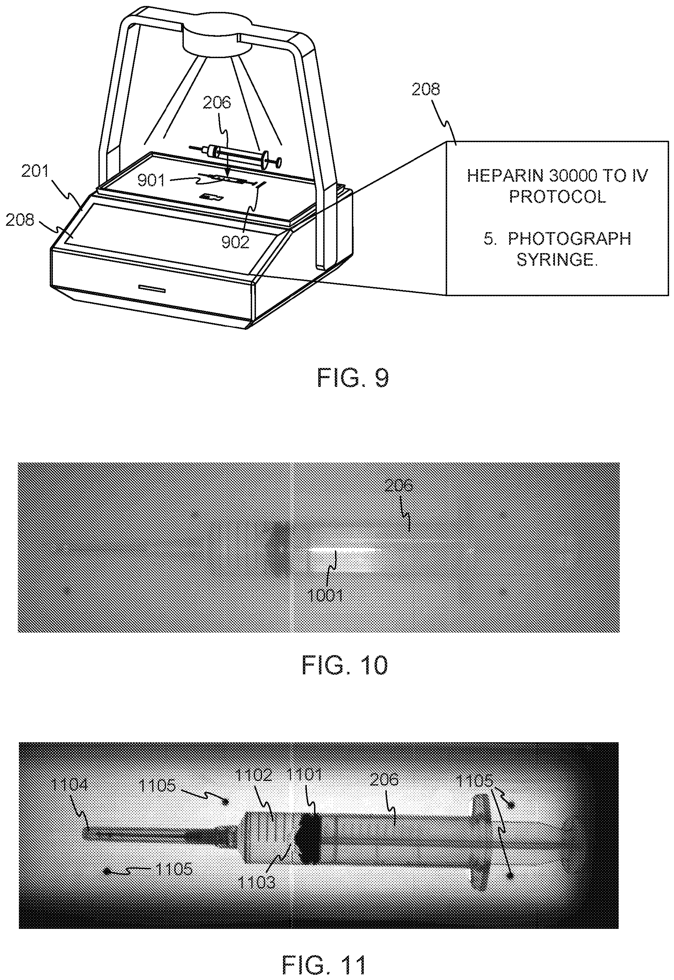

**FIG. 9** illustrates a fifth step, in which the filled syringe is photographed. For this purpose, tray **209** may include an icon **901** for placement of syringe **206,** and may include mechanical features facilitating correct placement and alignment of syringe **206** on tray **209,** for example a V-shaped trough, or a groove **902** shaped and sized to receive an edge of the barrel flange of syringe **206**. Other fiducial marks may be present as well.

Syringe **206** may be photographed using visible light camera **403,** but is preferably photographed using infrared camera **404.** **FIG. 10** shows a photograph as may be taken using visible light camera **403.** (Visible light camera **403** preferably has a field of view larger than shown in **FIG.** 10, but syringe **206** has been isolated from the larger view for ease of explanation.) While syringe **206** is readily visible in the photograph of FIG. 10, the photograph has been affected by glare spot **1001,** and may have been affected by ambient light sources that are not under the control of compounding assistance device **201.**

**FIG. 11** shows a photograph of syringe **206** as may be taken using infrared camera **404.** Tray **209** is not opaque to infrared radiation, so syringe **206** is backlit by infrared light source **302.** For example, tray **209** may be substantially transparent to infrared radiation, or may be translucent. In some embodiments, tray **209** may be made of polycarbonate or another suitable polymer or blend of polymers. Infrared camera **404** may have a wavelength-selective optical filter that passes infrared light to camera **404,** but blocks the visible spectrum. Thus, glare spots formed from visible light are excluded from the photograph of FIG. 11, resulting in greater clarity of features of syringe **206**.

Whichever kind of camera is used, compounding assistance device **201** can automatically analyze the resulting photograph for any of a number of purposes. For example (referring to **FIG.** 11), the position of the plunger **1101** of syringe **206** may be automatically recognized, and the amount of drawn liquid **1102** calculated based on the known dimensions of syringe **206**. In some embodiments, bubbles such as bubble **1103** may be detected and flagged if they are large enough to significantly affect the dose of medication being prepared. In some protocols, the weight of syringe **206** before and after drawing liquid from vial **204** may be used to verify that the correct amount of liquid was placed into syringe **206.** In that case, compounding assistance device **201** may also photograph syringe **206** at each weighing and analyze the photographs to detect whether syringe cap **1104** may have been mistakenly included in one weighing but not another. Fiducial marks **1105** on tray **209** are placed in known positions, and may be detected in the photograph and used to calibrate distances in the photograph.

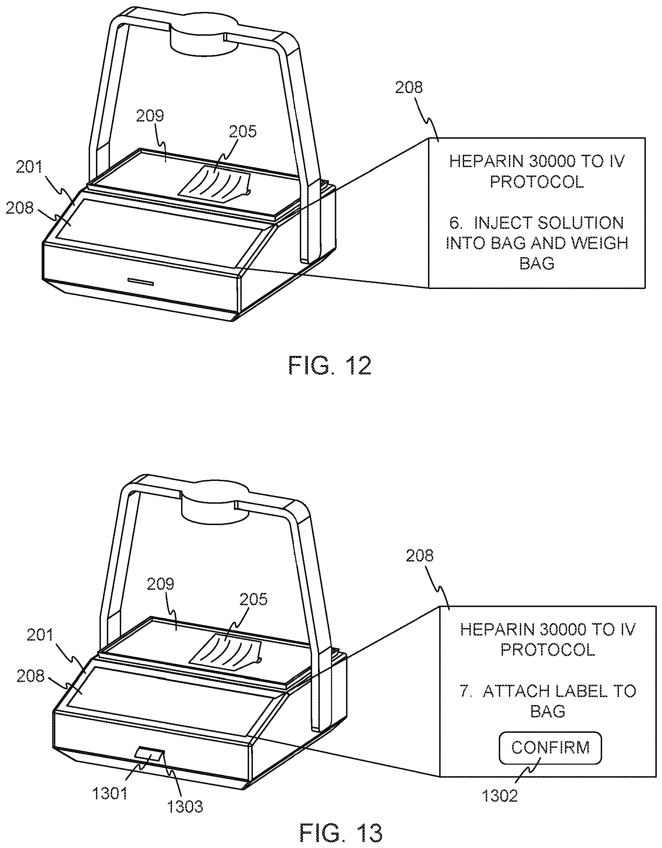

**FIG. 12** illustrates a sixth step, in which IV bag **205** is re-weighed after addition of solution from syringe **206**. Compounding assistance device **201** can compare the before and after weights of bag **205** to verify that the correct amount of Heparin solution was placed into bag **205.**

**FIG. 13** illustrates a seventh step, in which (presuming all of the checks in the system have verified that the compounding process was done correctly) compounding assistance device **201** prints a label **1301** using label printer **1303,** to be placed on bag **205,** and the user is prompted to adhere label **1301** to bag **205.** The finished medication can then be delivered to its point of use, and any consumable items disposed of, for example syringe **206.** The user may be asked to confirm **1302** that label **1301** has been affixed, using display **208.** In some embodiments, a final photograph of completed bag **205** may be taken for pharmacist review.

The compounding process described above is but one example, and many different compounding workflows may be implemented that have different steps, that use different medication containers, that collect different or additional information for process verification, or that differ in other ways from the example shown.

While the above example was shown in the context of compounding workstation **102a,** a similar process may be followed for compounding using a robotic compounder such as robotic compounder **103** shown in FIG. 1. A robotic compounder is a machine, usually enclosed, that use a robotic mechanism to handle vials, syringes, bags, and the like to prepare compounded medications. A robotic compounder may include a scale, one or more cameras, agitation devices, disposal ports, material and supply loading windows, and a delivery window for delivering a finished medication. Robotic compounders are not subject to human error in the compounding process, but include various weight and photographic checks on their work to guard against improper loading of materials, mechanical malfunctions, programming errors, and the like.

Whether compounding is done manually or robotically, the data collected during the compounding process is stored, for example on pharmacy server **101,** and can be reviewed by the responsible pharmacist. For example, the pharmacist can verify that the correct kind of vial containing the correct medication was identified by the barcode scan. The dosage can be verified by looking at the photograph of the syringe, the before and after weights of the vial, the before and after weights of the bag, or any combination of these. Any digital photographs taken during the compounding process may be made available for inspection by the pharmacist. For example, the pharmacist may look at a photograph such as the photograph of FIG. 11 to determine whether excessive bubbles may have been included in the liquid drawn into syringe **206.**

Upon completion of the compounding task, pharmacy server **101** may assign another compounding task to compounding station **102a,** and download another protocol to compounding assistance device **201** in accordance with the new task.

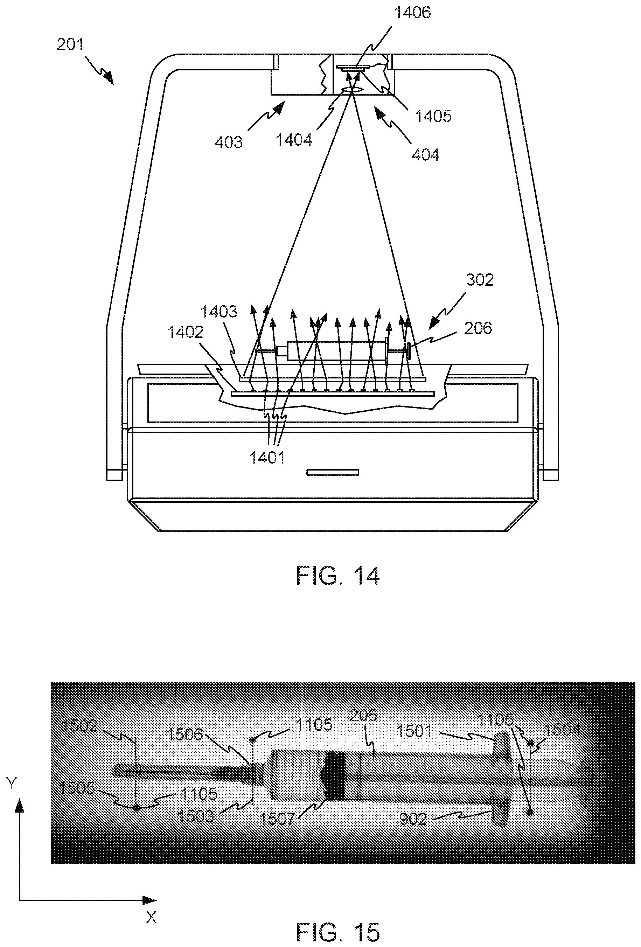

**FIG. 14** illustrates the arrangement of area light source **302** and infrared camera **404** of compounding assistance device **201,** in an embodiment of the invention.

In this example, area light source **302** includes an array of infrared light emitting diodes (LEDs) **1401** mounted to a circuit board **1402.** Light from infrared LEDs **1401** passes through a diffuser **1403,** and is scattered upward. Some of the light reaches a lens **1404** of infrared camera **404,** which forms an image onto an electronic array light sensor **1405,** which in turn is mounted on a printed circuit board **1406.** Printed circuit board **1406** may interface with a controller within compounding assistance device **201,** to receive signals for controlling electronic array light sensor **1405.**

Infrared LEDs **1401** may emit light in the near infrared wavelengths, for example between about 700 and 900 nanometers. In other embodiments, other wavelengths may be used. Diffuser **1403** provides a generally uniform backlight for items placed on area light source **302,** for example syringe **206.** Area light source **302** may be controlled by an electronic controller within compounding assistance device **201.** In some embodiments, tray **209** may be made of a diffusing material, and may be used in addition to or instead of diffuser **1403** to diffuse the light from area light source **302.**

In other embodiments, other kinds of light sources may be used, for example an edge-lit light guide plate having scattering features on one side. In this arrangement, light sources direct light into one or more edges of the light guide plate, and the light propagates by total internal reflection within the plate until it strikes one of the scattering features. Some of the scattered light is scattered out of the side of the plate opposite the scattering features. The scattering features are preferably distributed so that the intensity of the light exiting the plate is substantially uniform across the area of the plate. A diffuser may also be used to further diffuse the light exiting the plate, for additional uniformity in brightness.

Electronic array light sensor **1405** may be, for example, a charge coupled device (CCD) sensor, a complementary metal oxide semiconductor (CMOS) sensor, or another suitable kind of sensor. In general, such sensors exploit the property of some semiconductor materials that when the material is struck by light, free electrons are generated in proportion to the intensity of the light. The sensor is divided into specific light-sensitive areas called "pixels". To capture an image, the pixels are reset and then exposed to light for an exposure time. At the end of the exposure time, the amount of charge accumulated in each pixel is measured and converted to a numerical value. An array of these numerical values may be called a "digital image", with each value in the array representing the brightness of the light falling on the corresponding pixel.

In a CCD sensor, the accumulated charges are shifted off of the sensor to a charge amplifier, the output of which is digitized for each pixel. In a CMOS sensor, the accumulated charge can be read from each pixel directly, without shifting.

Electronic array light sensor **1405** may have any number of pixels sufficient to resolve features of interest at tray **209.** In some embodiments, electronic array light sensor **1405** may include an array 2560 X 1920 pixels, or about five megapixels. Other array sizes may be used in other embodiments. Electronic array light sensor **1405** is sensitive to light in the infrared wavelengths emitted by area light source **302.** For example, electronic array light sensor **1405** may be a silicon-based sensor sensitive to near infrared light. Infrared camera **404** may include an optical filter (not shown) that excludes other wavelengths. The optical filter may be, for example, a dichroic filter that passes light in the wavelengths of interest, but blocks light in other wavelengths, for example visible light.

As is explained above, infrared camera **404** can produce photographs of items on tray **209** that may be clearer in some aspects relevant to pharmaceutical compounding than photographs taken using visible light camera **403.** For example, glare spots caused by ambient room light can be largely eliminated. This clarity facilitates analysis of the digital photographs taken using infrared camera **404** for measurement and annotation that may be helpful to a reviewing pharmacist.

**FIG. 15** shows a photograph similar to the photograph of FIG. 11, taken using an infrared camera such as infrared camera **404,** and illustrating image analysis in accordance with embodiments of the invention. The controller within compounding assistance device **201** may "know" the relative positions of fiducial marks **1105,** as measured in image pixels, based on the known locations of fiducial marks **1105** on tray **209,** the number of pixels in electronic image sensor **1405,** and the magnification of the optical system including lens 1404. The controller can quickly locate the fiducial marks in the image by looking for a pattern of dark spots near the expected locations of the fiducial marks in the image. The pixel locations of the fiducial marks in the image may be recorded for reference.

As is visible in FIG. 15, barrel flange **1501** of syringe **206** has been placed in groove **902** of tray **209,** and thus barrel flange **1501** is precisely located with respect to fiducial marks **1105** in the "X" direction shown in FIG. 15. The controller may then query the brightness values of the pixels in the image near the fiducial marks, to locate edges of syringe **206** in the "Y" direction. For example, pixels along column lines **1502, 1503,** and **1504** may be analyzed, looking for abrupt light-to-dark and dark-to-light transitions that indicate the presence of edges of parts of syringe **206**.

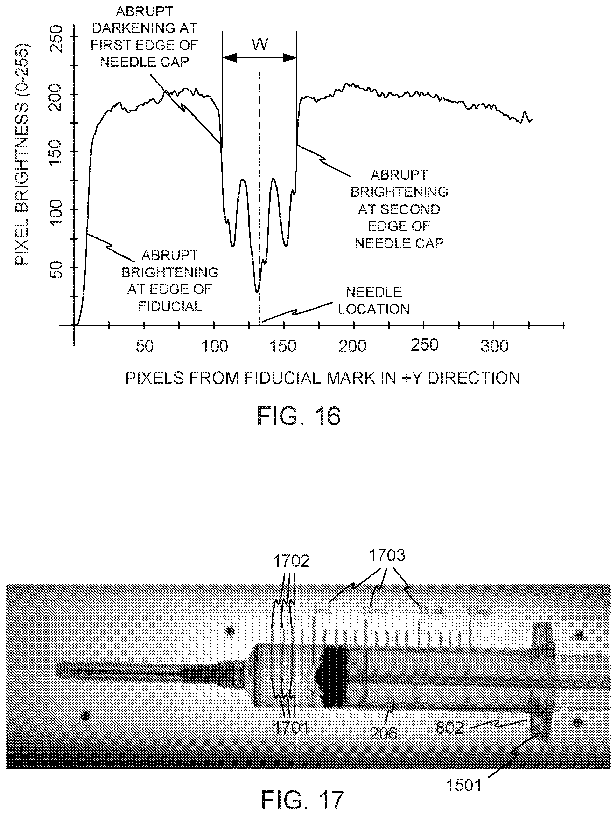

**FIG.** 16 illustrates an example of a trace of the brightness of pixels along column line **1502,** moving in the +Y direction from lower left fiducial mark **1505**. The transitions spanned by width W may be presumed to include be the needle of syringe **206**. If no drop in brightness is detected at the expected location of the needle, then the controller may consider that no needle is attached to syringe **206.** Presuming a drop is detected, then the centerline of the region spanned by width W may be presumed to be the centerline of the needle. The width W may be compared with known dimensions of the parts of syringe to determine whether a cap is present on the needle.

Referring again to FIG. 15, similar traces may be performed along other lines to detect the presence and size of a luer lock **1506,** or the presence and size of a plunger (not labeled) of syringe **206**. The detected dimensions may be compared with stored dimensions of standard syringes, so that the size of syringe **206** is automatically determined.

In other embodiments, other image processing techniques may be used to ascertain the location and size of a syringe from a digital image. For example, a correlation operation may be performed with a previously-prepared syringe photograph. The previous photograph may be compared with the current photograph in a number of orientations and positions, to find the location that best correlates with the syringe in the current photograph, to ascertain the location of the syringe in the current photograph. Fiducial marks **1105** may be found in this way as well. In other embodiments, a synthetic syringe image may be used in the correlation operation. Many other techniques are possible.

Once the size and location of syringe **206** are known in pixel space, the controller may annotate the digital image of the syringe, to assist the pharmacist in reviewing the compounding operation in which the image was taken.

In some embodiments, similar image processing techniques may be used to locate plunger **1507** in the digital image. Given the location of plunger **1507,** the location and orientation of syringe **206,** and the size of syringe **206**, an estimate of the volume of liquid in syringe **206** can be computed.

**FIG. 17** illustrates one way of annotating an infrared image, in accordance with embodiments of the invention. Once the size and location of syringe **206** have been ascertained, the size can be correlated to a standard syringe having pre-recorded measurements, including the locations of gradation marks **1701** on the syringe barrel indicating volumes of liquid in the syringe based on plunger position. While gradation marks **1701** are highly visible in FIG. 17, this may not always be the case. Depending on the positioning of the syringe on tray **209**, gradation marks **1701** may not be readily visible in any images. For example, syringe **206** may have been placed on tray **209** with gradation marks **1701** facing downward, or the liquid within syringe **206** may be opaque, hiding gradation marks **1701.**

Using the known size and position of syringe **206**, compounding assistance device **201** can annotate images taken by either of its cameras to enhance the readability of the plunger position. In FIG. 17, compounding assistance device has altered some of the pixels of the image to show lines **1702** corresponding to the computed locations of gradation marks **1701,** and has also added text **1703** showing the liquid volumes represented by lines **1702.** In other embodiments, other kinds of annotation may be provided, for example using different colors.

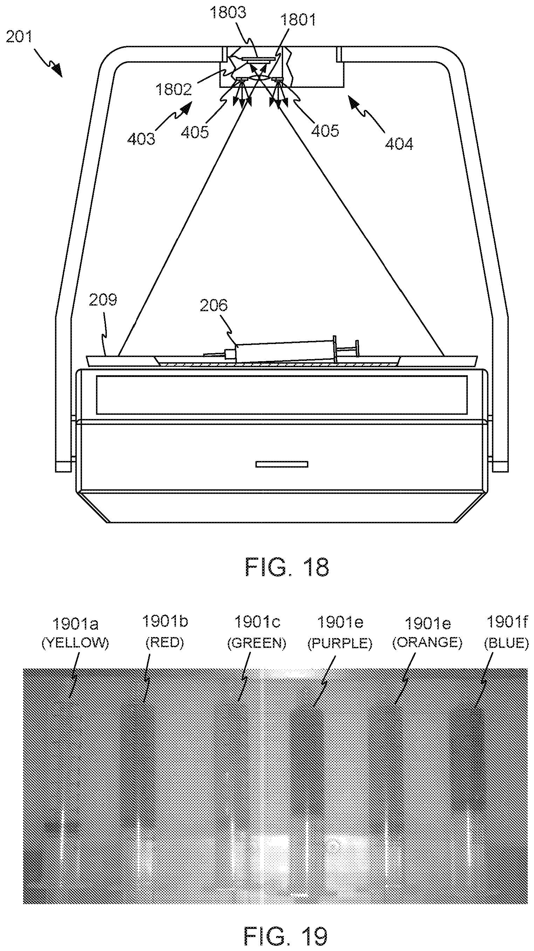

**FIG. 18** illustrates the arrangement of visible light camera **403** of compounding assistance device **201,** in an embodiment of the invention. Visible light camera **403** may be used to photograph items on tray **209**, for verification that the correct ingredients were used in a compounding task, for final verification that the resulting product looks as it should, or for other purposes. Visible light camera **403** may be a color camera, and may be especially useful for recording the color of a formulation as additional verification that the formulation is likely correct. (Example infrared camera **404** as described above cannot distinguish color due to the narrow band of infrared wavelengths it records and the lack of any color filters on its pixels.)

Visible light camera **403** includes a lens **1801** that focuses light received within its field of view onto an electronic sensor array light sensor **1802,** which is in turn mounted on a printed circuit board **1803.** Visible light camera **403** may include an optical filter (not shown) such as a dichroic filter that substantially prevents infrared wavelengths from reaching sensor **1802.** Light sources **405** may be used to supplement any ambient light illuminating tray **209.** For example, light sources **405** may be white LEDs directed at tray **209,** and controllable by the controller within compounding assistance device **201.**

Electronic array light sensor **1802** may be a CCD sensor, a CMOS sensor, or another suitable kind of sensor as described above, having enough pixels to resolve features of interest at tray **209.** For example, sensor **1802** may include an array 2560 X 1920 pixels, or about five megapixels. Other sensor sizes may be used. Sensor **1802** preferably includes color filters placed over individual pixels so that visible light camera **403** can record color images. For example, sensor **1802** may include red, green, and blue filters in the well-known Bayer pattern.

Visible light camera **403** and infrared camera **404** may be provided as pre-assembled camera modules that include standard interfaces for control by compounding assistance device **201.** Suitable camera modules are available from Basler AG of Ahrensburg, Germany, and IDS Imaging Development Systems GmbH of Obersulm, Germany.

**FIG. 19** shows an example photograph of a number of syringes **1901a-1901f** as may be taken by visible light camera **403** using light sources **405.** Each of syringes **1901a-1901f** contains fluid of a different color. Although the fluids in **FIG.** 19 are not necessarily pharmaceuticals, **FIG.** 19 illustrates that visible light camera **403** can distinguish a wide range of colors, and a photograph taken with visible light camera **403** may enable a pharmacist to verify that a compounded liquid is of an expected color, bolstering confidence that the compounding was done correctly, or to detect that a compounded liquid is not of the expected color, indicating that the compounding may not have been done correctly.

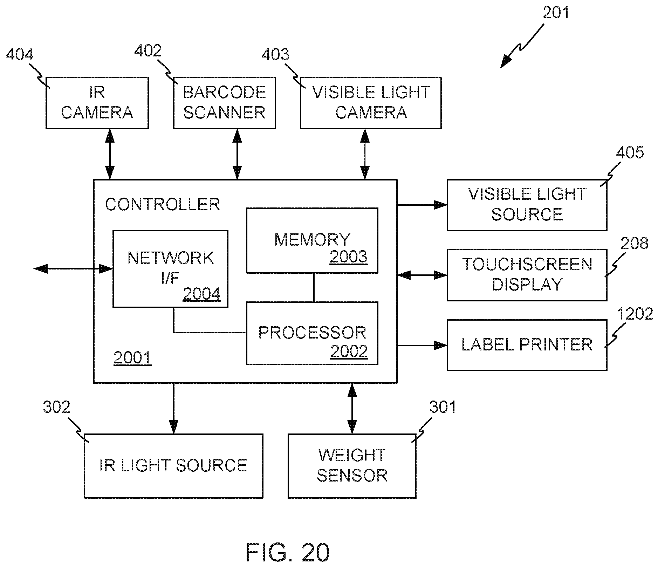

**FIG. 20** illustrates a simplified block diagram of compounding assistance device **201,** in accordance with embodiments of the invention. Compounding assistance device **201** includes a controller **2001** comprising a processor **2002**, memory **2003**, and a network interface **2004.** Memory **2003** may include dynamic memory, non-volatile memory, mass storage, or other kinds of memory in any suitable combination. Part of memory **2003** holds instructions for processor **2002** that, when executed, control the operation of compounding assistance device **201.** Other kinds of information may be stored in memory **2003** as well, for example working copies of digital images, temporary variables, and other kinds of information. Network interface **2004** allows controller **2001** to communication externally, for example with a server such as pharmacy server **101** described above.

Compounding assistance device **201** further includes infrared camera **404,** barcode scanner **402,** and visible light camera **403** as described above, all in communication with controller **2001** and under the control of controller **2001.** Infrared light source **302** and visible light source **405** are also under the control of controller **2001,** to be turned on and off at different times. In some embodiments, the intensity of the light produced by either or both light sources may be adjustable under the control of controller **2001.** Touchscreen display **208** can communicate information to a user of compounding assistance device **201,** and can receive instructions from the user.

Label printer **1303** receives commands and data from controller **2001** for the printing of labels. Weight sensor **301** provides signals to controller **2001** indicating the weight of tray **209** and any items on it.

Other architectures for compounding assistance device **201** may be used.

Additional information about compounding can be found in U.S. Patent Application No. 15/827,336 filed November 30, 2017 and titled "IV Compounding Systems and Methods,"

With the above understanding of an example compounding process, it will be recognized that many different compounding tasks are possible, using different numbers and combinations of ingredients. For example, different tasks may be defined for reconstituting and compounding medications received in powdered form, for medications to be delivered in a syringe for direct injection, for diluting medications received in concentrated form, or for other scenarios. More complex workflows may be designed for compounding multiple medications, for example placing multiple medications in a single IV drip bag for simultaneous delivery.

In some embodiments, pharmacy server **101** may oversee a number of pharmacies. For example, a single hospital may include a central pharmacy and one or more satellite pharmacies connected with pharmacy server **101.** In other embodiments, pharmacy server **101** may oversee multiple pharmacies in multiple sites. For example, a number of hospitals in a hospital group may share the services of pharmacy server **101.**

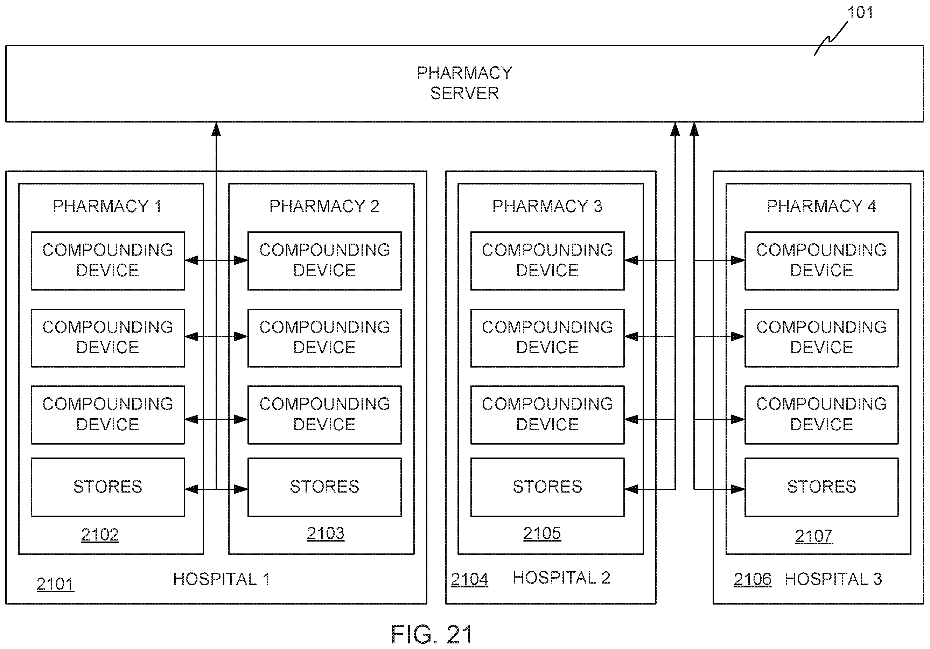

**FIG. 21** schematically shows one example arrangement of multiple pharmacies in multiple facilities, all in communication with pharmacy server **101,** in accordance with embodiments of the invention. First hospital **2101** includes two pharmacies **2102** and **2103.** Second hospital **2104** includes a pharmacy **2105,** and third hospital **2106** includes another pharmacy **2107.** Each pharmacy houses one or more compounding devices, and stores medicines and supplies. Pharmacy server **101** may be in communication with each of the pharmacies, even to the compounding device level.

Besides the basic requirements of safety and accuracy, the efficient operation of compounding pharmacies in a system such as the system shown in FIG. 21 is the subject of many considerations.

### Order Scheduling and Delivery Considerations

Different medication orders may have different priorities. For example, some orders designated as "stat" orders may require immediate compounding for delivery to a patient as soon as possible. Other orders may only need to be filled in due course, but of course should not be subject to unreasonable delay. For example, a "cart fill" order for a set of medications needed for a patient over the next few hours may be filled in due course. In some cases, medications may be prepared in batches, for example "premade" medications. Premade medications may be compounded formulations that are used commonly enough to justify accumulating a supply of them in anticipation of use, rather than compounding them for each individual order. Premades may be prepared in batches during off hours or at other times when compounding capacity is available in one or more of the pharmacies.

### Pharmaceutical Beyond Use Considerations

Medications typically have an allowable shelf life, often expressed as a "beyond use" date or time. The beyond use date is a date (or time) after which the medication is not to be used, so as to avoid any risk of loss of effectiveness or other problems with the medication. A single medication may have several beyond use dates or times, depending on its state of use. For example, a vial of medication newly received directly from the manufacturer may have a beyond use date weeks or months or more from the manufacturing date, so long as the vial remains unopened and is stored correctly. Once the vial is opened, it may have a viable shelf life of only hours or days, after which any unused amount should be discarded. And once part of the contents of the vial have been compounded into an IV drip bag, then the bag itself may have a beyond-use date or time measured from the time of compounding. When multiple medications or ingredients are used in a single compounding task, then the beyond use date or time of the compounded formulation may be the ingredient beyond use date or time nearest in the future.

### Inventory Management Considerations

Different cooperating pharmacies may have different levels of inventory at different times, which may be located at different physical locations. It may be desirable to cooperatively adjust the management of compounding tasks to efficiently share inventory, while avoiding excessive transportation of items between locations.

### Facility Utilization Considerations

The assignment of compounding tasks may be adjusted to allow for different operating times at different satellite pharmacies. Also, different pharmacies may have different equipment, some of which may be more or less suited to certain compounding tasks than equipment at a different pharmacy. All else being equal, certain tasks may be allocated to the pharmacy with the most suited equipment. In another example, different facilities may have different "off" hours than other facilities, when capacity may be available to perform batch compounding to frequently-used formulations.

### Compounding Management

In embodiments of the invention, pharmacy server **101** is programmed to assign compounding tasks to the various compounding devices, in light of the above and other considerations, with the goal of efficiently achieving the greatest feasible number of compounding task completions with the least feasible level of resource consumption, all on time and safely. Pharmacy server **101** may be programmed with many rules in pursuit of this goal.

The ability of server **101** to implement its efficiency improvements is enabled in part by the fact that server **101** assigns or "pushes" the compounding tasks to the compounding devices. In at least some prior arrangements, each compounder, upon completion of one compounding task, "pulls" the next available task from a queue. Because the individual compounders do not have visibility to the entire flow of orders or to the status of other pharmacies or facilities, a "pull" system cannot achieve the performance of a system having a global view and implementing a "push" system, as in embodiments of the invention.

For example, when a batch compounding task is planned, server **101** may recognize that two different compounding devices are available to work on the task, but that the two have different historical speeds of performance. In a hypothetical example, compounding workstation **102b** may be available, and may historically be able to complete one replication of the task at hand in 5 minutes, when used by the operator on duty. At the same time, robotic compounder **103** may be available, and may be able to complete one replication in 4 minutes. In this example, we suppose that 20 units of the compounded formulation are ordered. Server **101** may allocate the batch preparation to either or both of the two devices, depending on current needs. For example, in order to deliver the entire batch most quickly, server may assign portions of the task to both devices. However, if 10 units are assigned to each device, robotic compounder **103** will complete its 10 units in 40 minutes, while the operator at compounding station **102b** will require 50 minutes. Delivery of the entire order will have to wait on compounding station **102b** after robotic compounder **103** is finished.

Server **101** may instead assign unequal portions of the task to the two devices. In the above example, server **101** may assign 11 of the units to robotic compounder **103** and only 9 to compounding workstation **102b.** Robotic compounder **103** can complete its 11 units in 44 minutes, and the operator at station **102b** can complete his or her 9 units in 45 minutes. The overall 20 units are available for delivery in 45 minutes - 5 minutes sooner than if equal portions of the task were assigned to the two available devices. Within rounding error, server 101 in this example assigned portions of the task to the two devices such that the two portions were completed at the same time.

A large number of such strategies are possible.

In another example, compounding tasks may be reordered or assigned in groups to minimize the waste of pharmaceuticals. For example, a vial may contain 50,000 units of a pharmaceutical, and two medication orders may arrive at the pharmacy for compounding the pharmaceutical into IV drip bags - one containing 30,000 units of the pharmaceutical and one containing 20,000 units. If these two orders are simply assigned in sequence to the next available compounding devices, each of the devices may use a vial of the pharmaceutical, wasting 20,000 units at one device and 30,000 units at the other. Instead, server 101 may advance one of the others out of turn so that both can be assigned to the same compounding device and filled from the same 50,000 unit vial, resulting in no waste. Even if the two orders were assigned to the same compounding device but separated in time, waste could still occur if the shelf life of the opened vial is exceeded between tasks. The reordering of the tasks to occur in immediate sequence helps avoid this potential for waste as well.

Similarly, if several orders are received for compounding the same medication for different patients, these orders may be grouped and assigned to the same compounding device. In this way, supplies for the multiple orders may be pulled simultaneously and the compounding tasks may be performed without switchover time at the compounding device.

Server **101** may include the scheduled dosage times of the compounded formulations in its assignment rules as well. For example, in the scenario above, the two compounding tasks can also be scheduled so that both compounded IV bags can be delivered to their respective patients during the shelf life of the completed bags. In other words, it can also be important not to complete a compounding task too early, and risk the compounded formulation being unusable by the scheduled time of administration.

In another example, server **101** may assign compounding tasks to particular pharmacies, for example to avoid waste. For example, server **101** my keep or have access to inventory records of multiple pharmacies such as pharmacies **2102, 2103, 2105,** and **2107,** and may recognize that the stock of a particular pharmaceutical at one of the pharmacies is older than the stock at another of the pharmacies, and may be approaching its beyond use date. Server **101** may receive an order for compounding of the pharmaceutical, and may assign the compounding task to the pharmacy with the oldest viable stock, presuming that the compounded formulation can be delivered to the patient timely. In other embodiments, server **101** may consider the locations of the various pharmacies in this situation. For example, a "stat" order may not be assigned to a pharmacy in a different hospital than the patient for whom it is prescribed, because the time required to transport the compounded formulation back to the patient's hospital may be too long to meet the "stat" requirement. In this case, the order may be assigned for compounding in the same hospital as the patient, even though fulfilling the order in the same hospital may result in waste at another hospital as the same pharmaceutical goes past its beyond use date.

Similarly, server **101** may direct that a pharmaceutical be taken from smaller, more-expensive-per-unit vials than available larger, less-expensive-per-unit vials if the smaller vials are near their beyond-use date and may otherwise be wasted.

In another example, smaller, more-expensive-per-unit vials may be used if there is no convenient stock of the larger, less-expensive-per-unit vials, in order to meet compounding deadlines. In some embodiments, it may be acceptable to substitute an in-stock ingredient for a prescribed out-of-stock ingredient. For example, an IV drip bag with a different diluent than specified in the order may be substituted, preferably with pharmacist and/or physician approval. In some cases, server **101** may keep a list of approved substitutions.

Another management technique is the use of a premade formulation, when available, instead of performing a one-off compounding task. In other situations, a particular pharmacist or physician may specify that a fresh formulation be prepared in a specific case, even though a premade might be available.

Server **101** may consider patient demographics in the assignment of compounding tasks. For example, dose accuracy may be especially critical in pediatric care. When an order is received for a compounded formulation for a young child, a new formulation may be required to be prepared using a protocol with specific checks on the dose accuracy, even though a premade version of the same formulation is in stock, made according to a less stringent protocol and suitable for an adult patient. One way in which this situation may arise is an order for a pediatric dose in which the prescription translates into a dose volume of less than 2 ml. Because the measurement uncertainty of gravimetric verification may be as large as 10 percent or more at such small volumes, a protocol using photographic verification may be specified, showing the syringe plunger position, rather than using gravimetric verification. This decision could also be made based on the specific drug being prescribed. For example insulin is often prescribed in doses of less than 1 ml, such that photographic verification may be especially appropriate.

In the case of a particularly critical drug or dose size, server **101** may allow the specification of an in-workflow review, in which a second technician is called to review the work of the first and must enter their credentials for the prep to be approved.

In some embodiments, server **101** may consider the time of day, day of the week, day of the month, or other temporal data in assigning compounding tasks. In a simple example, a hospital main pharmacy may operate 24 hours per day, while a satellite pharmacy may close at night. Server **101** may assign a compounding task to the satellite pharmacy closest to the intended patient if the task can be completed during the operating hours of the satellite pharmacy. Otherwise, the task may be assigned to the main pharmacy, even though delivery will require more transportation time and effort. Similarly, tasks may be assigned to pharmacies in different facilities depending on operating hours.

The time of day, week, or month may be considered for other reasons as well. For example, a suburban pharmacy may historically have low demand for compounding on weekday afternoons as compared with a pharmacy in an urban setting. The suburban pharmacy may therefore be available to accept batch compounding tasks during weekday afternoons.

In some embodiments, server **101** may consider the kind of packages in which compounded medications will be delivered in assigning compounding tasks. For example, a series of similar tasks that are suitable for compounding by a robotic compounder may be grouped together and assigned to a particular robotic compounder, while "one-off" compounding tasks may be assigned to workstations with human operators. In some cases, certain delivery packages are simply more amenable to human compounding, for example ampoules, elastomeric pumps, and syringes of unusual size. Compounding tasks involving these delivery vehicles can be assigned to devices with human operators.

Preferably, server **101** can adapt in real time to changing conditions in the pharmacies within its purview. For example, in the event of an equipment breakdown or sudden operator illness, tasks scheduled for assignment to the failed compounding device can be reassigned to other devices.

The above strategies include further examples of the advantage of a "push" assignment system, as opposed to the traditional "pull" system. Pharmacy server **101** has visibility of inventories at the various compounding locations, and can "see" a broader range of incoming medication orders. It can therefor assign compounding tasks in such a way as to maximize the utilization of existing inventories, for example sending orders for the same medication to the same compounding device, so as to fully use a vial, or to use a medication near its beyond use date. A "pull" system in which each device or site has visibility only to its own location would not be able to achieve the same efficiencies.

In another example advantage, server **101** has visibility to the usage of compounding devices at multiple locations, and can assign tasks to take advantage of otherwise-idle devices or to offload devices that would otherwise be overloaded. A pull system would not be able to make these choices.

In another example advantage, the push system can assign portions of a repetitive batch compounding task to different devices, in proportions that maximize the overall speed of completion of the task.

The technical effect of such a system is more efficient utilization of assets and inventory that may be possible with a pull system, through load balancing and efficient inventory usage.

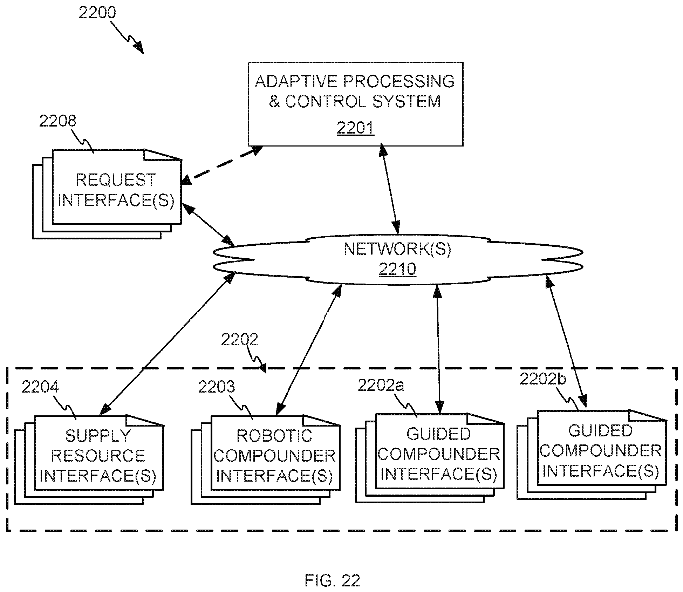

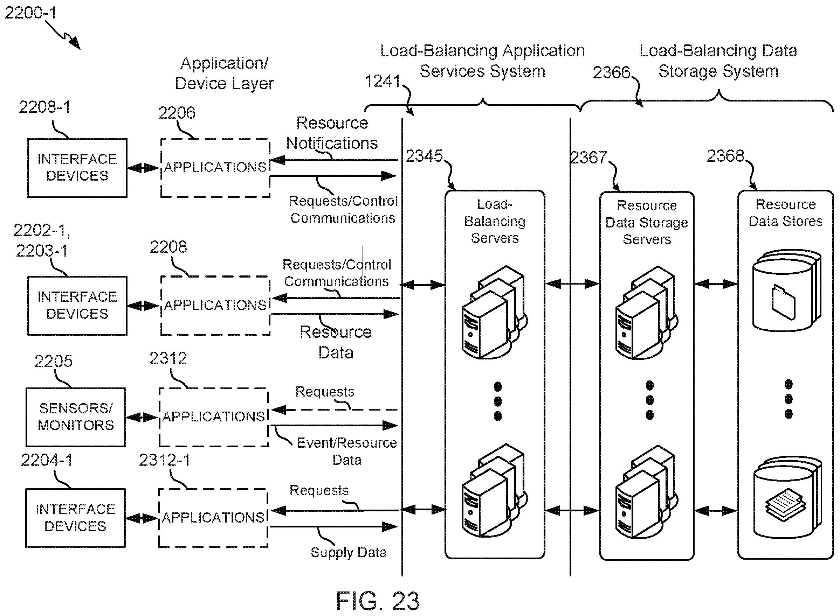

**FIGS. 22** **and** **23** illustrate additional technical details usable in embodiments, implemented as a resource control system **2200.** Resource control system **2200** may be used to implement the methods and techniques disclosed herein. **FIG. 22** depicts a high-level block diagram of the resource control system **2200,** in accordance with disclosed embodiments of the present disclosure. The resource control system **2200** may facilitate resource balancing and notifications relating to resource balancing based at least in part on resource capacities and assignments of operations to resources. In various embodiments, the resource control system **2200** may facilitate resource balancing, notifications, and control relating to resource balancing based at least in part on event recognition, as well. The resource control system **2200** may include an adaptive processing and control system **2201.** The adaptive processing and control system **2201** may aggregate and/or determine resource metrics and instantaneous resource state information to dynamically assess resource balance and demand. The adaptive processing and control system **2201** may intelligently manage loads based at least in part on resource models, which may define resources load processing specifications.

In various embodiments, the adaptive processing and control system **2201** may provide resource management control via onsite and/or offsite resource control gateways. As depicted, the resource control system **2200** may allow for interaction between two or more of an adaptive processing and control system **2201,** request interfaces **2208,** and a plurality of resources interfaces **2202,** which may include one or more supply resource interfaces **2204,** one or more robotic compounder interfaces **2203,** and one or more guided compounder interfaces **2202a, 2202b.** As disclosed herein, the one or more robotic compounder interfaces **2203** and one or more guided compounder interfaces **2202a, 2202b** may correspond to one or more compounding stations, which may include robotic compounders and guided compounding stations. As depicted, components of the resource control system **2200** may be communicatively coupled or couplable to one or more networks **2210.**

The one or more networks **2210** may be a suitable means to facilitate data transfer in the resource control system **2200** and could include multiple networks and/or network components. In various embodiments, the one or more networks **2210** may be implemented with, without limitation, one or more of the Internet, a wide area network (WAN), a local area network (LAN) such as one based on Ethernet, a virtual private network (VPN), an intranet, an extranet, Token-Ring and/or the like, an infra-red network, a wireless network (e.g., a network operating under Bluetooth^{®}, any of the Institute of Electrical and Electronics (IEEE) 802.11 suite of protocols, and/or any other wireless protocol), a wireless local area network (WLAN), a cellular network, such as through 1G, 3G, GSM (Global System for Mobile Communications), *etc.,* another wireless network, a gateway, a public switched telephone network (PSTN), common enterprise network, and/or any other appropriate architecture or system that facilitates the communication of signals, data, and/or message. In various embodiments, the one or more networks **2210** may transmit data using any suitable communication protocol(s), such as, without limitation, TCP/IP (transmission control protocol/Internet protocol), SNA (systems network architecture), IPX (Internet packet exchange), AppleTalk, and/or the like. In various embodiments, the one or more networks **2210** and its various components may be implemented using hardware, software, and communications media such wires, optical fibers, microwaves, radio waves, and other electromagnetic and/or optical carriers; and/or any combination of the foregoing and/or the like. In some embodiments, the network **2210** may include a telephone network that may be circuit switched, package switched, or partially circuit switched and partially package switched.

In various embodiments, the adaptive processing and control system **2201** may include a set of devices configured to process, transform, encode, translate, send, receive, retrieve, detect, generate, compute, organize, categorize, qualify, model, store, display, present, handle, or use information and/or data suitable for the embodiments described herein. The adaptive processing and control system **2201** may include a server system comprising one or more servers, each comprising one or more processors and memory. For example, one or more servers of the system **2201** may be used to store software programs and data. Software implementing the systems and methods described herein may be stored on storage media in the servers. Thus, the software may be run from the storage media in the servers. For example, memory of one or more of the servers may hold instructions that, when executed by one or more processors, cause a load-balancing server to perform its functions in accordance with embodiments of the invention. The storage may also store the records, protocols, and other information collected and generated in the operation of system **2200.** For the purposes of this disclosure, the term "memory" encompasses many different kinds of data storage devices and combinations of such devices, for example dynamic memory, static memory, volatile memory, nonvolatile memory, and mass storage such as magnetic or optical disk storage or tape storage. In various embodiments, software implementing the systems and methods described herein may be stored on storage media of other devices described herein.

In some embodiments, the resource control system **2200** may be a distributed system for implementing features of various embodiments disclosed herein. The resource control system **100** may include or otherwise interface with one or more interface devices **2208-1, 2202-1, 2203-1, 2204-1,** and/or **2205** depicted in FIG. 22. **FIG. 23** shows a diagram of a portion of an architecture stack of the system **2201,** in accordance with certain embodiments of the present disclosure. The architecture stack of FIG. 23 may also be used in implementations of the methods and techniques described herein. At least some of the interface devices **2208-1, 2202-1, 2203-1, 2204-1,** and/or **2205** may be configured to execute and operate a client application such as a web browser, proprietary client interface, application programming interface, and/or the like over one or more networks **2210.** Thus, the system **2201,** which may include a server system including one or more servers in some embodiments, may be communicatively coupled with remote interface devices **2208-1, 2202-1, 2203-1, 2204-1,** and/or **2205** via the network **2210.**

In various embodiments, the adaptive processing and control system **2201** may be adapted to run one or more services or software applications provided by one or more of the components of the system. In some embodiments, these services may be offered as web-based or cloud services or under a Software as a Service (SaaS) model to the users of remote interface devices **2208-1, 2202-1, 2203-1, 2204-1,** and/or **2205.** Users operating remote interface devices **2208-1, 2202-1, 2203-1, 2204-1,** and/or **2205** may in turn utilize one or more client applications to interact with the adaptive processing and control system **2201** to utilize the services provided by these components.

Software components of the resource control system **2200** may be implemented on the adaptive processing and control system **2201-**e.g., on one or more servers. In addition or in the alternative, one or more of the components of resource control system **2200** and/or the services provided by these components may be implemented by one or more of the interface devices **2208-1, 2202-1, 2203-1, 2204-1,** and/or **2205.** Users operating the interface devices **2208-1, 2202-1, 2203-1, 2204-1,** and/or **2205** may then utilize one or more client applications to use the services provided by these components. These components may be implemented in hardware, firmware, software, or combinations thereof. It should be appreciated that various different system configurations are possible, which may be different from the resource control system **2200.** The embodiment shown in the figure is thus one example for implementing certain embodiments and is not intended to be limiting.

Referring again to FIG. 22, the infrastructure **2201** may include one or more load-balancing servers, data acquisition servers, application servers, resource data management servers, and/or the like, one or more of which may include one or more load-balancing processors. The server system may be located remotely and/or locally with respect to one or more sites that generate, maintain, supply, and/or otherwise provide resources. The server system may acquire information, manage, and/or control site components of one or more sites, e.g., via resource interfaces **2202.** For example, resource descriptions, resource states, resource attributes, and/or the like to push and/or pulled from one or more sites via resource interfaces **2202.** Some embodiments may include implementing one or more on-site gateways providing information, management, and/or control of site components.

In various embodiments, one or more of the resource interfaces **2202** may allow for communication with one or more data sources, with the one or more interfaces **2202** configured to operate as one or more event monitors. The server system may correspond to, include, or otherwise utilize one or more event monitors to actively retrieve and/or otherwise receive data from one or more data sources. The one or more data sources may include any suitable source of data to facilitate embodiments disclosed further herein.

In various embodiments, the data from the one or more data sources may be retrieved and/or received by the adaptive processing and control system **2201** via the one or more event monitors through network(s) **2210** and/or through any other suitable means of transferring data. In some embodiments, the adaptive processing and control system **2201** and the data sources could use any suitable means for direct communication. According to certain embodiments, data may be actively gathered and/or pulled from one or more data sources, for example, by accessing a third party repository and/or by "crawling" various repositories. Certain data pulled and/or pushed from the one or more data sources may be transformed and the transformed data and/or other data generated as disclosed herein.

In various embodiments, one or more of the resource interfaces **2202** may include one or more gateways and/or application programming interfaces (APIs) that define protocols and routines for interfacing with the data sources. The APIs may specify application programming interface (API) calls to/from data source systems. Some embodiments may employ one or more web APIs. In some embodiments, the APIs may include a plug-in to integrate with an application of a data source system. The one or more of the resource interfaces **2202,** in some embodiments, could use a number of API translation profiles configured to allow interface with the one or more additional applications of the data sources to access data (e.g., a database or other data store) of the data sources. The API translation profiles may translate the protocols and routines of the data source system to integrate at least temporarily with the system and allow communication with the system by way of API calls. Data, as referenced herein, may correspond to any one or combination of raw data, unstructured data, structured data, information, and/or content which may include media content, text, documents, files, instructions, code, executable files, images, video, audio, and/or any other suitable content suitable for embodiments of the present disclosure.

According to certain embodiments, the adaptive processing and control system **2201** may include or provide a resource administratory platform. An administratory device may receive notifications and/or access the adaptive processing and control system **2201** via one or more request interfaces **2208.** In some embodiments, resource interfaces **2202** may interface with resource-controlling systems at remote sites.

In some embodiments, one or more request interfaces **2208** and/or a resource interfaces **2202** may include a web interface, which may, for example, allow for real-time and scheduled changing of resource assignments and schedules. The client interfaces **2205** and/or resource data interfaces **2202** may allow for transfer of and access to information in accordance with certain embodiments disclosed herein. In various embodiments, the request interfaces **2208** and/or resource interface(s) **2202** may include one or more suitable input/output modules and/or other system/devices operable to serve as an interface between users and the resource administratory platform. The request interfaces **2208** and/or resource interface(s) **2202** may facilitate communication over the network **2210** using any suitable transmission/ communication protocol and/or standard.

In various embodiments, the system **2201** may include, provide, and/or be configured for operation with the request interfaces **2208** and/or resource interface(s) **2202,** for example, by making available and/or communicating with one or more of a website, a web page, a web portal, a web application, a mobile application, enterprise software, and/or any suitable application software. In some embodiments, a request interfaces **2208** and/or resource interface(s) **2202** may include an API to interact with the interaction system **2201.**

In some embodiments, the request interfaces **2208** and/or resource interface(s) **2202** may include or work with an application made available to one or more interfaces, such as a mobile application as discussed herein. In some embodiments, the request interfaces **2208** and/or resource interface(s) **2202** may cause a web page to be displayed on a browser. The web page(s) may display output and receive input from a user (*e.g.,* by using Web-based forms, via hyperlinks, electronic buttons, *etc.*)*.* A variety of techniques can be used to create the web pages and/or display/receive information, such as JavaScript, Java applications or applets, JSON, dynamic HTML and/or AJAX technologies. Accordingly, the adaptive processing and control system **2201** may have web site(s)/portal(s) giving access to such information, such as an administratory portal. In various embodiments, a request interfaces **2208** and/or resource interface(s) **102** may include providing one or more display screen images that may each include one or more user interface elements. A user interface may include any text, image, and/or device that can be displayed on a display screen for providing information to a user and/or for receiving user input. A user interface may include one or more widgets, windows, dashboards, text, text boxes, text fields, tables, grids, charts, hyperlinks, buttons, lists, combo boxes, checkboxes, radio buttons, and/or the like.

Referring again to **FIG. 23****,** the depicted portion at least partially includes an application/device layer, as well as a load-balancing application services system **2341** and a load-balancing data management system **2366** of the system **2201.** In some embodiments, the load-balancing application services system **2341** may correspond at least partially to an interface layer and a load-balancing application services management layer. In some embodiments, the data storage system may correspond at least partially to a load-balancing data storage layer.

The load-balancing application services system **2341** may interface with the application/device layer **2210** and the load-balancing data storage system **2366.** In some embodiments, the load-balancing application services system **2341** may include at least part of the application/device layer **2210.** The load-balancing application services system **2341** could be a middle tier of the interaction system **2201** in some embodiments, with the load-balancing data storage system **2366** corresponding to a back-end in some embodiments.

In some embodiments, the load-balancing application services system **2341** and the load-balancing data storage system **2366** each may be or include a load-balancing server system **2345** and a resource data management server system **2367,** respectively, that include one or more servers. Other embodiments may include only a single server system. In some embodiments, the load-balancing application services system **2341** and the load-balancing data storage system **2366** may be integrated. In various embodiments, the server systems **2345, 2367** may include one or more computers, specialized server computers (including, by way of example, load-balancing servers, load control servers, other site control servers, data acquisition servers, application servers, data management servers, PC (personal computer) servers, UNIX^{®} servers, mid-range servers, mainframe computers, rack-mounted servers, etc.), server farms, server clusters, or any other appropriate arrangement and/or combination. In various embodiments, the server systems **2345, 2367** may be adapted to run one or more services, operations, processing, or software applications described herein. The server systems **2345, 2367** may also run any of a variety of additional server applications and/or mid-tier applications, including the examples disclosed above, for example, with respect to server **2312.**

In some embodiments, the server systems **2345, 2367** may include one or more applications to pull, receive, analyze, aggregate, and/or consolidate data feeds and/or event updates received from various data sources. As an example, data feeds and/or event updates may include, but are not limited to, application **2206, 2208, 2312,** and/or **2312-1** updates and/or data feeds, interfaces/devices updates and/or data feeds corresponding to the depicted feedback communications, which may include real-time events and/or data feeds related to sensor systems and/or components thereof, updates (real-time and/or otherwise) received from one or more third party information sources and/or continuous data streams, and/or the like. The server system **2345** may also include one or more applications to display the data feeds and/or real-time events via the interfaces/devices and/or devices internal to the interaction system **2201.**

The application services system **2341** and/or the data management system **2366** may also include one or more resource data storages **2368.** The resource data storages **2368** may include various forms of data storage including solid state storage, disk storage, databases (including relational, column, document, key-value and graph type databases) and cache. The resource data storages **2368** may reside in a variety of locations, such as on a non-transitory storage medium local to (and/or resident in) the server systems **2345, 2367** and/or remote from the server systems **2345, 2367** and in communication with the server systems **2345, 2367** via a network-based or dedicated connection. In certain embodiments, the resource data storages **2368** may reside in a storage-area network (SAN). Similarly, any necessary files for performing the functions attributed to the server systems **2345. 2367** may be stored locally on the server systems **2345, 2367** and/or remotely, as appropriate. In one set of embodiments, the resource data storages **2368** may include relational databases that are adapted to store, update, and retrieve data in response to SQL-formatted commands. It should be appreciated that information corresponding to the repositories may be stored elsewhere and/or in other ways, or may not be stored, depending on the implementations chosen. Likewise, while various segregations of data corresponding to the repositories are provided herein, it should be appreciated that such examples are non-limiting, and some or all the data may be handled in any suitable manner.

In certain embodiments, the adaptive system **2201** may be implemented in or with a distributed computing and/or cloud computing environment with a plurality of servers and cloud-implemented processing, memory, and data resources. Thus, with accretion of service information, the system may allow for scaling out with additional processing resources, server resources, data storage resources, data management resources, and the like. Some embodiments may use different types of servers to service different types of interface devices. The adaptive system **2201** may provision services facilitated by one or more components of the adaptive system **2201,** and, in some embodiments, one or more of the services may be offered as cloud services. A specific instantiation of a service provided by the adaptive system **2201** may be referred to herein as a service instance. In some examples, a service provided by the adaptive system **2201** may include control communications **2201,** which may correspond to any one or combination of communications disclosed herein such as communications to effect resource assignment, resource assignment such as assignment nullification, modification, reassignment, etc.

In the illustrated embodiment, one or more interface devices may be used by users to interact with the adaptive system **2201.** Although only a limited number of the interface devices is shown, any number of interface devices may be supported. In various embodiments, the interface devices may correspond to the request interfaces **2205** and/or resource interfaces **2202.** In various embodiments, the interface devices may include site component controllers and/or site components as disclosed further herein.

According to a first embodiment, a system for compounding of medications comprises a plurality of compounding devices, each of the plurality of compounding devices selected from the group of compounding devices consisting of a compounding assistance device, a robotic compounder, and a hazardous drug robotic compounder, and each of the plurality of compounding devices being computerized. The system further comprises a central server computer, and each of the plurality of compounding devices is in bidirectional communication with the central server computer via an electronic network. The central server computer and the plurality of compounding devices are configured to cooperatively receive, at the central server computer, a plurality of requests, at least some of which require the compounding of one or more medications; push, by the central server computer via the electronic network, assignments of respective compounding tasks to the plurality of compounding devices; and perform or guide, using the compounding devices, the respective directed compounding operations. The central server computer is configured to assign respective compounding tasks to the plurality of compounding devices in accordance with a set of rules designed to promote efficient use of compounding resources and avoid waste.

According to a second embodiment, the plurality of compounding devices in the first embodiment reside within a single pharmacy.

According to a third embodiment, the plurality of compounding devices in the first embodiment are distributed among multiple pharmacies within a single facility.

According to a fourth embodiment, the plurality of compounding devices in the first embodiment are distributed among multiple facilities.

According to a fifth embodiment, the central server computer in any of the above embodiments is configured to assign respective compounding tasks to the plurality of compounding devices in accordance with a rule that considers the physical locations of the plurality of compounding devices.

According to a sixth embodiment, the set of rules in any of the above embodiments is configurable.

According to a seventh embodiment, the set of rules in any of the above embodiments comprises one or more rules for reordering or grouping requests so as to avoid waste.

According to an eighth embodiment, the central server computer in any of the above embodiments is configured to assign respective portions of a batch compounding task to at least two of the plurality of compounding devices, wherein the respective portions of the batch compounding task assigned to each of the at least two compounding devices are selected based at least on part on performance records of the at least two compounding devices, such that the portions of the batch compounding task assigned to the at least two compounding devices are expected to be completed at the same time.

According to a ninth embodiment, compounding tasks are assigned in any of the above embodiments based at least in part on a beyond use date or time of a particular pharmaceutical.

According to a tenth embodiment, compounding tasks in any of the above embodiments are assigned based at least in part on one or more of a time of day, day of the week, or day of the month at which a particular compounding task is to be performed.

According to an eleventh embodiment, at least one batch compounding task is assigned in any of the above embodiments in a way that utilizes a compounding device for preparation of a batch of compounded formulation when the compounding device would otherwise be idle.

According to a twelfth embodiment, compounding tasks are assigned in any of the above embodiments based at least in part on patient demographics.

According to a thirteenth embodiment, at least some compounding tasks involving the same pharmaceutical are grouped and assigned in any of the above embodiments to be performed on a single compounding device.

According to a fourteenth embodiment, the central server computer is configured in any of the above embodiments to maintain records of the performance of each of the plurality of compounding devices, and compounding tasks are assigned to the compounding devices based at least in part on the records.

According to a fifteenth embodiment, a method of load balancing in a compounding system comprises receiving, at a central server computer, a plurality of requests, at least some of which require the compounding of one or more medications. The method further comprises monitoring the availability and performance of a plurality of compounding devices, each of the plurality of compounding devices selected from the group of compounding devices consisting of a compounding assistance device, a robotic compounder, and a hazardous drug robotic compounder, and each of the plurality of compounding devices being computerized. The method further comprises, for each of the requests requiring compounding: selecting one of the plurality of compounding devices to which to assign the task of compounding the medication, the selection being performed in accordance with a set of rules designed to promote efficient use of compounding resources and avoid waste; transmitting an electronic message assigning the task of compounding the medication to the selected compounding device; and performing or guiding, using the selected compounding device, the assigned compounding task.

According to a sixteenth embodiment, the plurality of compounding devices in the fifteenth embodiment all reside in a single facility.

According to a seventeenth embodiment, the plurality of compounding devices in the fifteenth embodiment are distributed among multiple facilities.

According to an eighteenth embodiment, for at least a particular one of the requests in any of the fifteenth to seventeenth embodiments, the selected compounding device is a robotic compounder, and the method comprises, performing the assigned compounding task using the robotic compounder.

According to a nineteenth embodiment, the method of any of the fifteenth to eighteenth embodiments further comprises tracking inventory levels of medicines at one or more locations, and selecting one of the plurality of compounding devices to which to assign a particular task comprises selecting the compounding device based at least in part on the inventory levels.

In the claims appended hereto, the term "a" or "an" is intended to mean "one or more." The term "comprise" and variations thereof such as "comprises" and " comprising," when preceding the recitation of a step or an element, are intended to mean that the addition of further steps or elements is optional and not excluded. It is to be understood that any workable combination of the elements and features disclosed herein is also considered to be disclosed.

## Claims

1. A system for compounding of medications, the system comprising:
a plurality of compounding devices, each of the plurality of compounding devices selected from the group of compounding devices consisting of a compounding assistance device, a robotic compounder, and a hazardous drug robotic compounder, and each of the plurality of compounding devices being computerized;
a first compounding assistance device;
a central server computer, wherein each of the plurality of compounding devices is in bidirectional communication with the central server computer via an electronic network;
wherein the central server computer and the plurality of compounding devices are configured to cooperatively:
receive, at the central server computer, a plurality of requests, at least some of which require the compounding of one or more medications;
push, by the central server computer via the electronic network, assignments of respective compounding tasks to the plurality of compounding devices, wherein the central server computer is configured to assign respective compounding tasks to the plurality of compounding devices in accordance with a set of rules designed to promote efficient use of compounding resources and avoid waste; and
perform or guide, using the compounding devices, the respective directed compounding operations.
wherein the first compounding assistance device comprises a tray (209) which provides a carrier for holding an item, e.g. a syringe, an IV bag or a vial, which item can be weighed or photographed while being held by the tray, wherein a V-shaped trough is formed into the tray,
the first compounding assistance device further comprising an area light source (302) which is an infrared light panel arranged to illuminate a portion of the tray (209), a gantry (401) spanning the tray, and an infrared camera (404) positioned on the gantry.

2. The system of claim 1, wherein the plurality of compounding devices reside within a single pharmacy.

3. The system of claim 1, wherein the plurality of compounding devices are distributed among multiple pharmacies within a single facility.

4. The system of claim 1, wherein the plurality of compounding devices are distributed among multiple facilities.

5. The system of any of claims 1-4, wherein the central server computer is configured to assign respective compounding tasks to the plurality of compounding devices in accordance with a rule that considers the physical locations of the plurality of compounding devices.

6. The system of any of claims 1-4, wherein the set of rules is configurable.

7. The system of any of claims 1-4, wherein the set of rules comprises one or more rules for reordering or grouping requests so as to avoid waste.

8. The system of any of claims 1-4, wherein the central server computer is configured to assign respective portions of a batch compounding task to at least two of the plurality of compounding devices, wherein the respective portions of the batch compounding task assigned to each of the at least two compounding devices are selected based at least on part on performance records of the at least two compounding devices, such that the portions of the batch compounding task assigned to the at least two compounding devices are expected to be completed at the same time.

9. The system of any of claims 1-4, wherein compounding tasks are assigned based at least in part on a beyond use date or time of a particular pharmaceutical.

10. The system of any of claims 1-4, wherein compounding tasks are assigned based at least in part on one or more of a time of day, day of the week, or day of the month at which a particular compounding task is to be performed.

11. The system of any of claims 1-4, wherein at least one batch compounding task is assigned in a way that utilizes a compounding device for preparation of a batch of compounded formulation when the compounding device would otherwise be idle.

12. The system of any of claims 1-4, wherein compounding tasks are assigned based at least in part on patient demographics.

13. The system of any of claims 1-4, wherein at least some compounding tasks involving the same pharmaceutical are grouped and assigned to be performed on a single compounding device.

14. The system of any of claims 1-4, wherein the central server computer is configured to maintain records of the performance of each of the plurality of compounding devices, and wherein compounding tasks are assigned to the compounding devices based at least in part on the records.

15. A method of load balancing in a compounding system, the method comprising:
receiving, at a central server computer, a plurality of requests, at least some of which require the compounding of one or more medications;
monitoring the availability and performance of a first compounding assistance device comprising a tray (209) which provides a carrier for holding an item, e.g. a syringe, an IV bag or a vial, which item can be weighed or photographed while being held by the tray, wherein a V-shaped trough is formed into the tray, and monitoring the availability and performance of a plurality of compounding devices, each of the plurality of compounding devices selected from the group of compounding devices consisting of a compounding assistance device, a robotic compounder, and a hazardous drug robotic compounder, and each of the plurality of compounding devices being computerized; the first compounding assistance device further comprising an area light source (302) which is an infrared light panel arranged to illuminate a portion of the tray (209), a gantry (401) spanning the tray, and an infrared camera (404) positioned on the gantry, and, for each of the requests requiring compounding:
selecting one of the plurality of compounding devices to which to assign the task of compounding the medication, the selection being performed in accordance with a set of rules designed to promote efficient use of compounding resources and avoid waste;
transmitting an electronic message assigning the task of compounding the medication to the selected compounding device; and
performing or guiding, using the selected compounding device, the assigned compounding task.

16. The method of claim 15, wherein the plurality of compounding devices all reside in a single facility.

17. The method of claim 15, wherein the plurality of compounding devices are distributed among multiple facilities.

18. The method of any of claims 15-17, wherein for at least a particular one of the requests, the selected compounding device is a robotic compounder, and the method comprises, performing the assigned compounding task using the robotic compounder.

19. The method of any of claims 15-17, further comprising tracking inventory levels of medicines at one or more locations, wherein selecting one of the plurality of compounding devices to which to assign a particular task comprises selecting the compounding device based at least in part on the inventory levels.

20. The method of any of claims 15-19, wherein monitoring the availability and performance of a plurality of compounding devices occurs via at least one event monitor.

21. The method of any of claims 15-20, further comprising: receiving the plurality of requests at the central server computer from a plurality of request interfaces.

22. The method of any of claims 15-21, wherein transmitting the electronic message assigning the task of compounding the medication to the selected compounding device comprises: transmitting the electronic message to a resource interface associated with the selected compounding device.

23. The method of any of claims 15-22, wherein transmitting the electronic message assigning the task of compounding the medication to the selected compounding device comprises: pushing the electronic message assigning the task of compounding the medication to the selected compounding device.

24. The method of any of claim 15-23, the first compounding assistance device comprising:
an infrared light panel located under the tray and configured to illuminate at least a portion of the tray with infrared light; and
an infrared camera located above the tray and configured to capture an image of the tray.

## Patentansprüche

1. System zum Compoundieren von Medikamenten, wobei das System umfasst:
mehrere Compoundiervorrichtungen, wobei jede der mehreren Compoundiervorrichtungen aus der Gruppe von Compoundiervorrichtungen ausgewählt wird, die aus einer Compoundierunterstützungsvorrichtung, einem Roboter-Compounder und einem Roboter-Compounder für gefährliche Arzneimittel besteht und jede der mehreren Compoundiervorrichtungen computergestützt ist;
eine erste Compoundierunterstützungsvorrichtung;
einen zentralen Servercomputer, wobei jede der mehreren Compoundiervorrichtungen über ein elektronisches Netzwerk in bidirektionaler Kommunikationsverbindung mit dem zentralen Servercomputer steht;
wobei der zentrale Servercomputer und die mehreren Compoundiervorrichtungen dazu ausgelegt sind, hierzu zusammenzuwirken:
Empfangen, an dem zentralen Servercomputer, mehrerer Anfragen, von denen wenigstens einige die Compoundierung eines oder mehrerer Medikamente erfordert;
Push-Übertragung, durch den zentralen Servercomputer, über das elektronische Netzwerk, von Zuweisungen jeweiliger Compoundieraufgaben an die mehreren Compoundiervorrichtungen, wobei der zentrale Servercomputer dazu ausgelegt ist, den mehreren Compoundiervorrichtungen jeweilige Compoundieraufgaben gemäß einem Satz von Regeln zuzuweisen, die dafür konzipiert sind, eine effiziente Nutzung von Compoundierressourcen zu fördern und Ausschuss zu vermeiden; und
Durchführen oder Leiten, unter Verwendung der Compoundiervorrichtungen, der jeweiligen gelenkten Compoundieroperationen.
wobei die erste Compoundierunterstützungsvorrichtung eine Ablage (209) umfasst, die einen Träger zum Halten eines Gegenstands, z. B. einer Spritze, eines IV-Beutels oder einer Phiole, bereitstellt, wobei dieser Gegenstand gewogen oder fotografiert werden kann, während er von der Ablage gehalten wird, wobei eine V-förmige Mulde in der Ablage gebildet ist,
wobei die erste Compoundierunterstützungsvorrichtung ferner eine Flächenlichtquelle (302), bei der es sich um eine Infrarotlichttafel handelt, die so angeordnet ist, dass sie einen Teil der Ablage (209) beleuchtet, eine Gantry (401), welche die Ablage umspannt, und eine Infrarotkamera (404), die an der Gantry positioniert ist, umfasst.

2. System nach Anspruch 1, wobei sich die mehreren Compoundiervorrichtungen in einer einzelnen Apotheke befinden können.

3. System nach Anspruch 1, wobei die mehreren Compoundiervorrichtungen auf mehrere Apotheken innerhalb einer einzelnen Einrichtung verteilt sind.

4. System nach Anspruch 1, wobei die mehreren Compoundiervorrichtungen auf mehrere Einrichtungen verteilt sind.

5. System nach einem der Ansprüche 1-4, wobei der zentrale Servercomputer dazu ausgelegt ist, den mehreren Compoundiervorrichtungen jeweilige Compoundieraufgaben gemäß einer Regel zuzuweisen, welche die physischen Standorte der mehreren Compoundiervorrichtungen berücksichtigt.

6. System nach einem der Ansprüche 1-4, wobei der Regelsatz konfigurierbar ist.

7. System nach einem der Ansprüche 1-4, wobei der Regelsatz eine oder mehrere Regeln zum Neuordnen oder Gruppieren von Anfragen umfasst, um Ausschuss zu vermeiden.

8. System nach einem der Ansprüche 1-4, wobei der zentrale Servercomputer dazu ausgelegt ist, jeweilige Teile einer Chargencompoundieraufgabe wenigstens zwei der mehreren Compoundiervorrichtungen zuzuweisen, wobei die jeweiligen Teile der Chargencompoundieraufgabe, die jeder der wenigstens zwei Compoundiervorrichtungen zugewiesen werden, wenigstens zum Teil basierend auf Leistungsaufzeichnungen der wenigstens zwei Compoundiervorrichtungen zugewiesen werden, sodass die Teile der Chargencompoundieraufgabe, die den wenigstens zwei Compoundiervorrichtungen zugewiesen werden, erwartungsgemäß zur gleichen Zeit abgeschlossen werden.

9. System nach einem der Ansprüche 1-4, wobei Compoundieraufgaben wenigstens teilweise basierend auf einem Verfallsdatum oder einer Verfallszeit eines bestimmten Pharmazeutikums zugewiesen werden.

10. System nach einem der Ansprüche 1-4, wobei Compoundieraufgaben wenigstens teilweise basierend auf einem oder mehreren von einer Tageszeit, einem Wochentag oder einem Tag des Monats, an dem eine bestimmte Compoundieraufgabe durchgeführt werden soll, zugewiesen werden.

11. System nach einem der Ansprüche 1-4, wobei wenigstens eine Chargencompoundieraufgabe in einer Weise zugewiesen wird, die eine Compoundiervorrichtung zur Vorbereitung einer Charge einer Compound-Formulierung nutzt, wenn die Compoundiervorrichtung andernfalls ungenutzt wäre.

12. System nach einem der Ansprüche 1-4, wobei Compoundieraufgaben wenigstens teilweise basierend auf Patientendemografien zugewiesen werden.

13. System nach einem der Ansprüche 1-4, wobei wenigstens einige Compoundieraufgaben, die dasselbe Pharmazeutikum beinhalten, gruppiert und so zugewiesen werden, dass sie auf einer einzelnen Compoundiervorrichtung durchgeführt werden.

14. System nach einem der Ansprüche 1-4, wobei der zentrale Servercomputer dazu ausgelegt ist, Aufzeichnungen der Leistung jeder der mehreren Compoundiervorrichtungen zu speichern, und wobei Compoundieraufgaben den Compoundiervorrichtungen wenigstens teilweise basierend auf den Aufzeichnungen zugewiesen werden.

15. Verfahren zum Lastausgleich in einem Compoundiersystem, wobei das Verfahren umfasst:
Empfangen, an einem zentralen Servercomputer, mehrerer Anfragen, von denen wenigstens einige die Compoundierung eines oder mehrerer Medikamente erfordert;
Überwachen der Verfügbarkeit und Leistung einer ersten Compoundierunterstützungsvorrichtung, umfassend eine Ablage (209), die einen Träger zum Halten eines Gegenstands, z. B. einer Spritze, eines IV-Beutels oder einer Phiole, bereitstellt, wobei dieser Gegenstand gewogen oder fotografiert werden kann, während er von der Ablage gehalten wird, wobei eine V-förmige Mulde in der Ablage gebildet ist, und Überwachen der Verfügbarkeit und Leistung mehrerer Compoundiervorrichtungen, wobei jede der mehreren Compoundiervorrichtungen aus der Gruppe von Compoundiervorrichtungen ausgewählt wird, die aus einer Compoundierunterstützungsvorrichtung, einem Roboter-Compounder und einem Roboter-Compounder für gefährliche Arzneimittel besteht und jede der mehreren Compoundiervorrichtungen computergestützt ist; wobei die erste Compoundierunterstützungsvorrichtung ferner eine Flächenlichtquelle (302), bei der es sich um eine Infrarotlichttafel handelt, die so angeordnet ist, dass sie einen Teil der Ablage (209) beleuchtet, eine Gantry (401), welche die Ablage umspannt, und eine Infrarotkamera (404), die an der Gantry positioniert ist, umfasst, und, für jede der Anfragen, die eine Compoundierung erfordern:
Auswählen einer der mehreren Compoundiervorrichtungen, der die Aufgabe der Compoundierung des Medikaments zugewiesen werden soll, wobei die Auswahl gemäß einem Satz von Regeln durchgeführt wird, die dafür konzipiert sind, eine effiziente Nutzung von Compoundierressourcen zu fördern und Ausschuss zu vermeiden;
Übertragen einer elektronischen Nachricht, mit der die Aufgabe des Compoundierens des Medikaments der ausgewählten Compoundiervorrichtung zugewiesen wird; und
Durchführen oder Leiten, unter Verwendung der ausgewählten Compoundiervorrichtung, der zugewiesenen Compoundieraufgabe.

16. Verfahren nach Anspruch 15, wobei sich die mehreren Compoundiervorrichtungen alle in einer einzelnen Einrichtung befinden.

17. Verfahren nach Anspruch 15, wobei die mehreren Compoundiervorrichtungen auf mehrere Einrichtungen verteilt sind.

18. Verfahren nach einem der Ansprüche 15-17, wobei für wenigstens eine bestimmte der Anfragen die ausgewählte Compoundiervorrichtung ein Roboter-Compounder ist und das Verfahren das Durchführen der zugewiesenen Compoundieraufgabe unter Verwendung des Roboter-Compounders umfasst.

19. Verfahren nach einem der Ansprüche 15-17, ferner umfassend das Verfolgen von Lagerbeständen von Medikamenten an einem oder mehreren Standorten, wobei das Auswählen einer der mehreren Compoundiervorrichtungen, der eine bestimmte Aufgabe zugewiesen werden soll, das Auswählen der Compoundiervorrichtung wenigstens teilweise basierend auf den Lagerbeständen umfasst.

20. Verfahren nach einem der Ansprüche 15-19, wobei das Überwachen der Verfügbarkeit und Leistung mehrerer Compoundiervorrichtungen über wenigstens einen Ereignismonitor erfolgt.

21. Verfahren nach einem der Ansprüche 15-20, ferner umfassend: Empfangen der mehreren Anfragen an dem zentralen Servercomputer von mehreren Anfrageschnittstellen.

22. Verfahren nach einem der Ansprüche 15-21, wobei das Übertragen der elektronischen Nachricht, mit der die Aufgabe des Compoundierens des Medikaments der ausgewählten Compoundiervorrichtung zugewiesen wird, dies umfasst: Übertragen der elektronischen Nachricht an eine Ressourcenschnittstelle, die mit der ausgewählten Compoundiervorrichtung verknüpft ist.

23. Verfahren nach einem der Ansprüche 15-22, wobei das Übertragen der elektronischen Nachricht, mit der die Aufgabe des Compoundierens des Medikaments der ausgewählten Compoundiervorrichtung zugewiesen wird, umfasst: Push-Übertragung der elektronischen Nachricht, mit der die Aufgabe des Compoundierens des Medikaments der ausgewählten Compoundiervorrichtung zugewiesen wird.

24. Verfahren nach einem der Ansprüche 15-23, wobei die erste Compoundierunterstützungsvorrichtung umfasst:
eine Infrarotlichttafel, die sich unter der Ablage befindet und dazu ausgelegt ist, wenigstens einen Teil der Ablage mit Infrarotlicht zu beleuchten; und
eine Infrarotkamera, die sich über der Ablage befindet und dazu ausgelegt ist, ein Bild der Ablage zu erfassen.

## Revendications

1. Système de préparation de médicaments, le système comprenant:
une pluralité de dispositifs de préparation, chacun de la pluralité de dispositifs de préparation sélectionné dans le groupe de dispositifs de préparation étant constitué d'un dispositif d'aide à la préparation, d'un robot de préparation et d'un robot de préparation de médicaments dangereux, et chacun de la pluralité de dispositifs de préparation étant informatisé;
un premier dispositif d'aide à la préparation;
un ordinateur serveur central, dans lequel chacun de la pluralité de dispositifs de préparation est en communication bidirectionnelle avec l'ordinateur serveur central par l'intermédiaire d'un réseau électronique;
où l'ordinateur serveur central et la pluralité de dispositifs de préparation sont configurés pour, en coopération:
recevoir, au niveau de l'ordinateur serveur central, une pluralité de demandes, dont au moins certaines nécessitent la préparation d'un ou plusieurs médicaments;
pousser, par l'ordinateur serveur central, par l'intermédiaire du réseau électronique, des attributions de tâches de préparation respectives à la pluralité de dispositifs de préparation, l'ordinateur serveur central étant configuré pour attribuer des tâches de préparation respectives à la pluralité de dispositifs de préparation conformément à un ensemble de règles conçues pour favoriser une utilisation efficace des ressources de préparation et éviter le gaspillage; et
effectuer ou guider, à l'aide des dispositifs de préparation, les opérations de préparation dirigées respectives,
où le premier dispositif d'aide à la préparation comprend un plateau (209) qui fournit un support pour maintenir un article, par exemple une seringue, une poche pour perfusion intraveineuse ou une flacon, lequel article peut être pesé ou photographié tout en étant maintenu par le plateau, où une goulotte en forme de V est formée dans le plateau,
le premier dispositif d'aide à la préparation comprenant en outre une source de lumière de zone (302) qui est un panneau de lumière infrarouge agencé pour éclairer une partie du plateau (209), un portique (401) recouvrant le plateau, et une caméra infrarouge (404) positionnée sur le portique.

2. Système selon la revendication 1, dans lequel la pluralité de dispositifs de préparation résident dans une seule pharmacie.

3. Système selon la revendication 1, dans lequel la pluralité de dispositifs de préparation sont répartis entre de multiples pharmacies au sein d'une seule installation.

4. Système selon la revendication 1, dans lequel la pluralité de dispositifs de préparation sont répartis entre de multiples installations.

5. Système selon l'une quelconque des revendications 1-4, dans lequel l'ordinateur serveur central est configuré pour affecter des tâches de préparation respectives à la pluralité de dispositifs de préparation conformément à une règle qui considère les emplacements physiques de la pluralité de dispositifs de préparation.

6. Système selon l'une quelconque des revendications 1-4, dans lequel l'ensemble de règles est configurable.

7. Système selon l'une quelconque des revendications 1-4, dans lequel l'ensemble de règles comprend une ou plusieurs règles pour réordonner ou grouper des demandes de manière à éviter le gaspillage.

8. Système selon l'une quelconque des revendications 1-4, dans lequel l'ordinateur serveur central est configuré pour affecter des parties respectives d'une tâche de préparation de lots à au moins deux de la pluralité de dispositifs de préparation, où les parties respectives de la tâche de préparation de lots affectées à chacun des au moins deux dispositifs de préparation sont sélectionnées sur la base, au moins en partie, d'enregistrements de performances des au moins deux dispositifs de préparation, de telle sorte que les parties de la tâche de préparation de lots affectées aux au moins deux dispositifs de préparation soient censées être achevées en même temps.

9. Système selon l'une quelconque des revendications 1-4, dans lequel des tâches de préparation sont attribuées sur la base, au moins en partie, d'une date ou heure de dépassement d'utilisation d'un produit pharmaceutique particulier.

10. Système selon l'une quelconque des revendications 1-4, dans lequel des tâches de préparation sont attribuées sur la base, au moins en partie, d'un ou plusieurs éléments parmi un moment du jour, un jour de la semaine ou un jour du mois auquel une tâche de préparation particulière doit être exécutée.

11. Système selon l'une quelconque des revendications 1-4, dans lequel au moins une tâche de préparation de lots est attribuée d'une manière qui utilise un dispositif de préparation pour la préparation d'un lot de formulation composée lorsque le dispositif de préparation serait sinon inactif.

12. Système selon l'une quelconque des revendications 1-4, dans lequel des tâches de préparation sont attribuées sur la base, au moins en partie, de données démographiques du patient.

13. Système selon l'une quelconque des revendications 1-4, dans lequel au moins certaines tâches de préparation impliquant le même produit pharmaceutique sont regroupées et affectées pour être exécutées sur un dispositif de préparation unique.

14. Système selon l'une quelconque des revendications 1-4, dans lequel l'ordinateur serveur central est configuré pour maintenir des enregistrements des performances de chacun de la pluralité de dispositifs de préparation, et dans lequel des tâches de préparation sont attribuées aux dispositifs de préparation sur la base, au moins en partie, des enregistrements.

15. Procédé d'équilibrage de charge dans un système de préparation, le procédé comprenant les étapes suivantes:
recevoir, au niveau d'un ordinateur serveur central, une pluralité de demandes, dont au moins certaines nécessitent la préparation d'un ou plusieurs médicaments;
surveiller la disponibilité et les performances d'un premier dispositif d'aide à la préparation comprenant un plateau (209) qui fournit un support pour maintenir un article, par exemple une seringue, une poche pour perfusion intraveineuse ou un flacon, lequel article peut être pesé ou photographié tout en étant maintenu par le plateau, où une goulotte en forme de V est formée dans le plateau, et surveiller la disponibilité et les performances d'une pluralité de dispositifs de préparation, chacun de la pluralité de dispositifs de préparation étant choisi dans le groupe de dispositifs de préparation constitué d'un dispositif d'aide à la préparation, d'un robot de préparation et d'un robot de préparation de produits pharmaceutiques dangereux, et chacun de la pluralité de dispositifs de préparation étant informatisé; le premier dispositif d'aide à la préparation comprenant en outre une source lumineuse de zone (302) qui est un panneau lumineux infrarouge agencé pour éclairer une partie du plateau (209), un portique (401) enjambant le plateau, et une caméra infrarouge (404) positionnée sur le portique, et, pour chacune des demandes nécessitant une préparation:
sélectionner l'un de la pluralité de dispositifs de préparation auquel attribuer la tâche de préparation du médicament, la sélection étant effectuée selon un ensemble de règles destinées à favoriser une utilisation efficace des ressources de préparation et à éviter le gaspillage;
transmettre un message électronique attribuant la tâche de préparation du médicament au dispositif de préparation sélectionné; et
exécuter ou guider, à l'aide du dispositif de préparation sélectionné, la tâche de préparation affectée.

16. Procédé selon la revendication 15, dans lequel la pluralité de dispositifs de préparation résident tous dans une seule installation.

17. Procédé selon la revendication 15, dans lequel la pluralité de dispositifs de préparation sont répartis entre de multiples installations.

18. Procédé selon l'une quelconque des revendications 15-17, dans lequel, pour au moins une demande particulière des demandes, le dispositif de préparation sélectionné est un préparateur robotisé, et le procédé comprend d'exécuter la tâche de préparation attribuée en utilisant le préparateur robotisé.

19. Procédé selon l'une quelconque des revendications 15-17, comprenant en outre de suivre les niveaux d'inventaire des médicaments au niveau d'un ou de plusieurs emplacements, où la sélection de l'un de la pluralité de dispositifs de préparation auquel attribuer une tâche particulière comprend la sélection du dispositif de préparation sur la base, au moins en partie, des niveaux d'inventaire.

20. Procédé selon l'une quelconque des revendications 15-19, dans lequel la surveillance de la disponibilité et des performances d'une pluralité de dispositifs de préparation survient par l'intermédiaire d'au moins un moniteur d'événements.

21. Procédé selon l'une quelconque des revendications 15-20, comprenant en outre: la réception de la pluralité de demandes au niveau de l'ordinateur serveur central à partir d'une pluralité d'interfaces de demande.

22. Procédé selon l'une quelconque des revendications 15-21, dans lequel la transmission du message électronique attribuant la tâche de préparation du médicament au dispositif de préparation sélectionné comprend: la transmission du message électronique à une interface de ressource associée au dispositif de préparation sélectionné.

23. Procédé selon l'une quelconque des revendications 15-22, dans lequel la transmission du message électronique attribuant la tâche de préparation du médicament au dispositif de préparation sélectionné comprend: la poussée du message électronique attribuant la tâche de préparation du médicament au dispositif de préparation sélectionné.

24. Procédé selon l'une quelconque des revendications 15-23, le premier dispositif d'aide à la préparation comprenant:
un panneau lumineux infrarouge situé sous le plateau et configuré pour éclairer au moins une partie du plateau avec de la lumière infrarouge; et
une caméra infrarouge située au-dessus du plateau et configurée pour capturer une image du plateau.
